**Europäisches Patentamt**

(19) **European Patent Office**

**Office européen des brevets**

(11) Publication number : **0 172 865 B2**

(12) # NEW EUROPEAN PATENT SPECIFICATION

(45) Date of publication of the new patent specification : **01.06.94 Bulletin 94/22**

(51) Int. Cl.[5] : **C12N 15/30, A61K 39/015, C12Q 1/68, G01N 33/569**

(21) Application number : **85901031.6**

(22) Date of filing : **20.02.85**

(86) International application number : **PCT/GB85/00072**

(87) International publication number : **WO 85/03725 29.08.85 Gazette 85/19**

(54) DNA SEQUENCES, RECOMBINANT DNA AND PROCESSES FOR PRODUCING ANTIGENS OF PLASMODIUM FALCIPARUM.

(30) Priority : **20.02.84 GB 8404378**

(43) Date of publication of application : **05.03.86 Bulletin 86/10**

(45) Publication of the grant of the patent : **20.03.91 Bulletin 91/12**

(45) Mention of the opposition decision : **01.06.94 Bulletin 94/22**

(84) Designated Contracting States : **AT BE CH DE FR GB LI LU NL SE**

(56) References cited :
GB-A- 2 096 893
GB-A- 2 099 300
NATURE, vol. 289, 22 January 1981, L.H. Perrin et al.:"Inhibition of P. falciparum growth in human erythrocytes by monoclonal antibodies", pp. 301-303
PROCEEDINGS OF THE NATIONAL ACADEMY OF SCIENCES, USA, vol. 80, June 1983; D.J. Kemp et al.:"Expression of Plasmodium falciparum blood-stage antigens in Escherichia coli:Detection with antibodies from immune humans", pp. 3787-3791
NATURE, vol. 306, 22/29 December 1983; R.L. Coppel et al.:"Isolate-specific S-antigens of plasmodium falciparum contains a repeated sequence of eleven amino acids", pp. 751-754
PROCEEDINGS OF THE NATIONAL ACADEMY OF SCIENCES, USA, vol. 81, June 1984; M.J. McGarvey et al.:"Identification and expression in Escherichia coli of merozoite stage-specific genes of the human malarial parasite Plasmodium falciparum", pp. 3690-3694
CHEMICAL ABSTRACTS, vol. 97, no. 9, 30 August 1982 (Columbus, Ohio, US); M. Goman et al.:"The establishment of genomic DNA libraries for the human malaria parasite Plasmodium falciparum and identification of individual clones by hybridization", p. 152, abstract no. 67168g

(73) Proprietor : **BEHRINGWERKE Aktiengesellschaft Postfach 1140 D-35001 Marburg (DE)**

(72) Inventor : **MACH, Bernard 45, route de Pregny CH-1292 Chambésy (CH)**
Inventor : **PERRIN, Luc 12, avenue Peschier CH-1211 Geneva 4 (CH)**
Inventor : **McGARVEY, Michael 11, parc des Mayens 1218-Grand-Saconnex Geneva (CH)**
Inventor : **CHEUNG, Andrew Hoh Cazenove Hall Wellesley College Wellesley, MA 02181 (US)**
Inventor : **SHAW, Alan 19, quai du Cheval CH-1227 Geneva (CH)**

EP 0 172 865 B2

(56) References cited :

**BIOLOGICAL ABSTRACTS/RRM, Biosciences Information Service (Philadelphia, PA, US); J. Scaife et al.:"Application of recombinant DNA techniques to the human malaria parasite Plasmodium falciparum. 12th annual UCLA Symposium on Molecular Biology of Host-Parasite interactions"**

**Chemical Abstracts, vol.101, no.17, 22 October 1984 (Columbus, Ohio, US), K.G. Odink et al.:"Expression of cloned cDNA for a major surface antigen of Plasmodium falciparum merozoites, p. 166, abstract no. 145092v**

**Nature, vol. 311, 27 September 1984, R. Hall et al.:"Major surface antigen gene of a human malaria parasite cloned and expressed in bacteria", pp. 379-382**

**Nature, vol. 311, 27 September 1984, M. Koenen et al.:"Human antisera detect a Plasmodium falciparum genomic clone encoding a nonpeptide repeat", pp. 382-385**

**Nature, vol. 311, 27 September 1984, M. Koenen et al.:"Human antisera detect a Plasmodium falciparum genomic clone encoding a nonpeptide repeat", pp. 382-385**

**Nature, vol. 294, 26 November 1981, A.A. Holder, R.R. Freeman.:"Immunization against blood-stage rodent malaria using purified parasite antigens", pp. 361-364**

**J.Exp.Med., November 1983, R.R. Freeman, A.A. Holder:"Surface antigens of malaria merozites", vol. 158, pp. 1647-1653**

**Annals of Tropical Medicine and Parasitology, Vol. 77, no. 1, 95-96 (1983), Holder and Freeman**

**J. Exp. Med., vol. 156, pp. 1528-1538 (Nov. 1982), Holder & Freeman**

**Molecular and Biochemical Parasitology, 7 (1983), 159-172, Silveira & Mercereau-Puijalon**

**Molecular and Biochemical Parasitology, 10 (Jan. 1984), 55-66, Odink et al.**

**Proc. Natl. Acad. Sci., USA, vol. 80, pp. 3787-3791, June 1983, Kemp et al.**

**Bull. World Health Organization, 57 suppl. 1, 53-61 (1979), Reese et al.**

**Proc. Natl. Acad. Sci., USA, vol. 77, pp. 3695-3699, June 1980, Kilejian**

**Cell, vol. 16, 443-452, Febr. 1979, St. John and Davis**

## Description

TECHNICAL FIELD OF INVENTION

This invention relates to DNA sequences, recombinant DNA molecules and processes for producing late schizont-merozoite stage specific antigens of the human malarial parasite plasmodium falciparum. More particularly, this invention relates to late schizont-merozoite stage specific antigens of the human malarial parasite Plasmodium falciparum, methods of producing them in transformed hosts and methods using them and compositions containing them for protecting humans against that parasitic infection. It also relates to methods and means of using those antigens and their antibodies in the diagnosis of that parasitic infection.

BACKGROUND ART

Malaria remains a major cause of morbidity and mortality in many parts of the world [3rd Annual Report on the Special Program For Research and Train ing in Tropical Diseases, WHO, Geneva (1979)]. Almost one third of the world's population is exposed to the risk of infection. There are an estimated 150 million cases of malaria per year and in Africa alone the annual mortality, mainly among children, is about 1 million [J.A. Deans and S. Cohen, "Immunology Of Malaria", Ann. Rev. Microbiol., 37, pp. 25-49 (1963); A. Noguer et al., WHO Chron., 32, pp. 9-17 (1978)]. Accordingly, malaria constitutes a major constraint on economic progress in tropical and subtropical areas of the world.

Malaria is caused by the parasite plasmodium. More than 100 plasmodial species have been described that cause malaria in a wide range of vertebrates. These species, however, display a narrowly-defined host specificity. For example, only four species infect man: P. falciparum, P. vivax, P. malariae and P. ovale. P. falciparum is by far the most lethal of these species. There is also a high degree of antigenic heterogeneity within a particular plasmodium species [E.g., R. L. Coppel et al., "Isolate-Specific S-Antigen Of Plasmodium Falciparum Contains A Repeated Sequence Of Eleven Amino Acids", Nature, 306, pp. 751-56 (1983); R.F. Anders et al., Characterization Of An S-Antigen Synthesized By Several Isolates Of Plasmodium Falciparum". Proc. Natl. Acad. Sci. USA, 80, pp. 6652-56 (1983)].

Global eradication of malaria is beset by many practical difficulties. These problems have been compounded by the increasing resistance of mosquitos to insecticides and by the appearance of strains of malarial parasites that are resistant to the more commonly used chemotherapeutic agents, including 4-amino quinolines, like chloroquine. Accordingly, the preparation of a vaccine against malaria has been, and continues to be, a long sought after goal.

The preparation of such vaccines is beset by several difficulties. For example, preparation of antigenic compounds from parasites grown in culture is very difficult, particularly on the large scale necessary for a useful vaccine. In addition, the life cycle of the malarial parasite presents a variety of potential targets for the action of specific immune processes. For example, protective immunity can operate against two distinct development stages of the parasite -- the sporozoites and the asexual blood stages [G.V. Brown et al., "Target Antigens Of Purified Human Immunoglobulins which Inhibit Growth Of Plasmodium Falciparum In Vitro", Nature, 297, pp. 591-93 (1982)].

Sporozoites are introduced into the body by the bite of the mosquito. The asexual blood stages are largely responsible for the clinical symptoms of malaria. They involve maturation through a number of development stages called ring, trophozoite and schizont Finally, at the end of the asexual cycle, mature schizonts rupture the red blood cells and merozoites are released into the circulation. The released merozoites then attach to specific receptors on other red cell membranes to initiate invasion. They also differentiate into male and female gametocytes which may be ingested by mosquitos and sexually reproduced by them to produce sporozoites for subsequent infection.

Because the sporozoite is the first form of the parasite presented to the immune system, it is an attractive target for a malaria vaccine. However, the sporozoite's time of transit between its injection with the mosquito bite and the beginning of the malarial blood stage in the liver is very short. Moreover, only one sporozoite reaching the liver may be sufficient to cause the disease. Accordingly, the level of circulating antibodies necessary to insure 100% killing would be exceedingly difficult to maintain.

A vaccine against the merozoite stage should induce the type of immunity found in adults in endemic areas. The sporozoite stage would be unaffected, but the first issue of merozoites from the liver would be attacked by the vaccine-induced antibodies of the immune system. Indeed, this burst of merozoites might serve as a natural booster to that immune response. A gametocyte vaccine, on the other hand, has the theoretical advantage of breaking the chain of transmission, but that vaccine has the possible ethical disadvantage of not protecting in a direct manner the individual being vaccinated. Accordingly, we believe the optimal vaccine should be a blood stage vaccine.

It has been shown that antigens of the asexual blood stage, and in particular of schizonts and merozoites, play a major role in protective immunity against malaria. For example, immunoglobulins from immune donors recognize schizont-merozoite specific antigens [L.H. Perrin and R. Dayal., Immunol. Rev., 61, pp. 245-69 (1982); G.V. Brown et al., Nature, 297, pp. 591-93 (1982); J.H.L. Playfair, Bull. WHO, 57, pp. 245-46 (1979)]. Immune serum and monoclonal antibodies to schizont-merozoites have also demonstrated an inhibitory activity against parasite growth in vitro. [Brown et al. supra ; Playfair, supra ; L.H. Perrin et al., Nature, 289, pp. 301-O2 (1981); L. Schofield et al., Infect. Immun., 38,pp. 893-97 (1982)].

Moreover, the Perrin et al. monoclonal antibodies react with plasmodium isolates from various parts of the world and inhibit their growth. It has also been demonstrated that monkeys, the conventional malarial test animal, after injection with defined merozoite-specific polypeptides of P. falciparum are protected from a lethal intravenous challenge with parasites [L.H. Perrin et al., Clin. Exp. Immunol, (1984); B. Berkli et al., Abstract Sixth Int. Cong. of Parasitology, Calgary (1984)]. Significantly, these protective polypeptides, of molecular weights 200, 140, 82 and 41 k daltons, were the same polypeptides as those immunoprecipitated by the Perrin et al. monoclonal antibodies that have demonstrated an inhibitory activity against growth of P. falciparum in vitro [L.H. Perrin and R. Dayal, supra ; L.H. Perrin et al., supra ]. Accordingly, additional supplies of these polypeptides and others having the same protective activity are needed to develop an effective vaccine against malaria. However, there are presently no sources of these polypeptides available that enable such large scale production.

Various proteins and antigens have been produced in appropriate hosts using recombinant DNA technology [e.g., S. Nagata et al., "Synthesis in E.coli Of a Polypeptide With Human Leukocyte Interferon Activity", Nature, 284, pp. 316-20 (1980) (IFN-$\alpha$), C.J. Burrell et al., "Expression In Escherichia coli Of Hepatitis B Virus DNA Sequences Cloned In Plasmid pBR322", Nature, 279, pp. 43-48 (1979); M. Pasek et al., "Hepatitis B Virus Genes And Their Expression In E.coli", Nature, 282, pp. 575-79 (1979) (hepatitis B viral antigens), and H. Kupper et al., "Cloning Of cDNA Of Major Antigen Of Foot And Mouth Disease Virus And Expression In E.coli", Nature, 289, 555-559 (1981) (FMD viral antigens)].

Certain malarial antigens have also been stated to have been cloned in E.coli [K.G. Odink et al., "Cloning Of Malarial Genes Coding For High Molecular Weight Antigens: Isolation Of Fragments Of Plasmodium Falciparum Genes Coding For Proteins Of 145,000 Molecular Weight", Mol. Biochem. Parasite, 10, pp. 55-66 (1984) (a "145,000 molecular weight antigen")]. Other have been reported to have been expressed in E.coli. These include a malarial sporozoite surface antigen from P. Knowlesi [J. Ellis et al., "Cloning And Expression In E.coli Of The Malarial Sporozoite Surface Antigen Gene From Plasmodium Knowlesi", Nature, 302, pp. 536-38 (1983): G.N. Godson et al., "Identification And Chemical Synthesis Of A Tandemly Repeated Immunogenic Region Of Plasmodium Knowlesi Circumsporoite Protein", Nature, 305, pp. 29-33 (1983)]. This antigen is reported to be characterized by a DNA sequence having a 12 times repeated 36-base pair unit. Moreover, a chemically synthesized peptide having the amino acid sequence of this repeated subunit immunoreacted with a monoclonal antibody raised against the CS protein of the H strain of P. Knowlesi. The sporozoite antigen reported above also is said to include amino acid sequences on either side of the repeated subunits that are neither partial nor degenerate copies of that repeated subunit. The immunogenic activity of these sequences was not investigated.

In addition, a group of unidentified "blood stage" antigens have been stated to have been cloned and expressed in E.coli [D.J. Kemp et al., "Expression Of Plasmodium Falciparum Blood-Stage Antigens In Escherichia Coli: Detection With Antibodies From Immune Humans". Proc. Natl. Acad. Sci. USA, 80, pp 3787-91 (1983)].

There is no description in Kemp et al. that the blood stage antigens are specific to any development stage of the asexual blood antigens. Instead. they are said to be derived from RNA from a mixture of all stages of the erythrocytic cycle. There is also no disclosure in Kemp et al. than any of these antigens are specifically recognized by a monoclonal antibody that is specific to one of the polypeptides that has been shown to protect monkeys from P.falciparum infection.

One of the Kemp et al. antigens has been reported to be isolate-specific and to contain a repeated sequence of 11 amino acids [R.L. Coppel et al., "Isolate-Specific S-Antigen Of Plasmodium Falciparum Contains A Repeated Sequence Of Eleven Amino Acids". Nature , 306. pp. 751-56 (1983)]. Antibodies raised by this antigen are said to be reactive with the mature trophozoite and more intensely with the schizont stage of the asexual blood cycle. They did not react with immature ring forms of that cycle. Again, there is no description that the selected antigen is specifically recognized by a monoclonal antibody that is specific to one of the polypeptides that affords parasite immunity in monkeys [L.H Perrin et al., supra ]. Moreover, the selected antigen is said to be isolate specific. Therefore, antibodies to it are not crossreactive with antigens from P.falciparum species from different areas of the world.

## DISCLOSURE OF THE INVENTION

The present invention solves the problems referred to by isolating various families of DNA sequences that code for stage specific late schizontmerozoite antigens. These sequences are then used to transform appropriate hosts and to produce the antigens coded for by them.

The antigens produced by this invention are recognized by human malaria infected serum and a pool of monoclonal sera ($\alpha$200K, $\alpha$140K, $\alpha$82K and $\alpha$41K) specific to the demonstrated malarial protective antigens. The antigens of this invention also raise antibodies in animals that immunoprecipitate at least one of the antigens (200 kd) that have been demonstrated to protect monkeys from malaria. Moreover, these antibodies react in an immunofluorescence assay with P. falciparum isolates from a wide variety of geographic locations.

Accordingly, the antigens of this invention are useful in globally effective protective compositions and methods against malaria. They, or their antibodies, are also useful in diagnostic means and methods to detect the presence of malarial infections in various blood samples.

It will be appreciated from the foregoing that a basic aspect of this invention is the selection and characterization of various families of DNA sequences that code on expression for stage-specific late schizont-merozoite antigens of P.falciparum. These families of DNA sequences are selected from the group consisting of families V14, G2, R14, D4, X11, M2, T5, M7, V4, D8, I4 and E8. Each family is characterized by cross-hybridization to a particular cDNA clone of the same name which is produced in accordance with this invention. Accordingly, additional members of these particular families may be selected from collections of DNA sequences from various sources, including natural, synthetic or semi-synthetic sources, and more particularly from genomic banks of DNA sequences from the various plasmodia species that infect humans.

The DNA sequences of this invention are further characterized in that they permit the expression of late schizont-merozoite stage specific antigens at high level for use in globally effective protective and diagnostic applications with respect to malarial infections.

## BRIEF DESCRIPTION OF THE DRAWINGS

Figure 1 is an autoradiograph of an SDS-PAGE of $35_s$ methionine-labelled proteins from the various asexual erthrocytic stages of P.falciparum: rings (R), trophozoites (T), early schizonts (S) and mature segmented schizonts (Seg). The arrows indicate the late schizont-merozoite (SEG) stage specific polypeptide bands.

Figure 2 is an autoradiograph of a gridded filter containing once-selected late schizont-merozoite stage specific clones -- (A): screened with a late schizont-merozoite enriched probe, and (B): screened with a ring enriched probe.

Figure 3 is an autoradiograph of a Northern blot hybridization of ring stage specific RNA (R) and late schizont-merozoite stage specific RNA (M) with two clones (A and B) selected in accordance with this invention to contain late schizont-merozoite stage specific cDNA sequences. The arrows indicate the positions of 28S and 18S human liver ribosomal RNAs and "28S" and "18S" P.falciparum ribosomal RNAs.

Figure 4 is an autoradiograph of "relatedness" hybridizations with probes prepared from the inserts of late schizont-merozoite stage specific clones D4(A) and V14(B).

Figure 5 is the nucleotide sequence, and derived amino acid sequence, of the malarial insert of p31-1.

Figure 6 is the nucleotide sequence, and derived amino acid sequence, of the malarial insert of pFCC-1.

## BEST MODE OF CARRYING OUT THE INVENTION

In order that the invention herein described may be more fully understood, the following detailed description is set forth.

In this description the following terms are employed:

Nucleotide - A monomeric unit of DNA or RNA consisting of a sugar moiety (pentose), a phosphate, and a nitrogenous heterocyclic base. The base is linked to the sugar moiety via the glycosidic carbon (1′ carbon of the pentose) and that combination of base and sugar is called a nucleoside. The base characterizes the nucleotide. The four DNA bases are adenine ("A"), guanine ("G"), cytosine ("C"), and thymine ("T"). The four RNA bases are A, G, C, and uracil ("U").

DNA Sequence - A linear array of deoxy nucleotides connected one to the other by phosphodiester bonds between the 3′ and 5′ carbons of adjacent pentoses.

Codon - A DNA sequence of three nucleotides (a triplet) which encodes through its mRNA an amino acid, a translation start signal or a translation termination signal. For example, the nucleotide triplets TTA, TTG, CTT, CTC, CTA and CTG encode for the amino acid leucine ("Leu"), TAG, TAA and TGA are translation stop signals and ATG is a translation start signal.

5

Reading Frame - The grouping of codons during the translation of mRNA into amino acid sequences. During translation the proper reading frame must be maintained. For example, the DNA sequence GCTGGTTGTAAG may be expressed in three reading frames or phases, each of which affords a different amino acid sequence:

GCT GGT TGT AAG - Ala-Gly-Cys-Lys
G CTG GTT GTA AG - Leu-Val-Val
GC TGG TTG TAA G - Trp-Leu-(STOP)

Polypeptide - A linear array of amino acids connected one to the other by peptide bonds between the α-amino and carboxy groups of adjacent amino acids.

Genome - The entire DNA of a cell or a virus. It includes inter alia the gene coding for the polypeptides of the substance, as well as operator, promoter and ribosome binding and interaction sequences, including sequences such as the Shine-Dalgarno sequences.

Gene - A DNA sequence which encodes through its template or messenger RNA ("mRNA") a sequence of amino acids characteristic of a specific polypeptide from mRNA.

Expression - The process undergone by a gene to produce a polypeptide. It is a combination of transcription and translation.

Plasmid - A nonchromosomal double-stranded DNA sequence comprising an intact "replicon" such that the plasmid is replicated in a host cell. When the plasmid is placed within a unicellular organism, the characterisitics of that organism may be changed or transformed as a result of the DNA of the plasmid. For example, a plasmid carrying the gene for tetracycline resistance (Tet^R) transforms a cell previously sensitive to tetracycline into one which is resistant to it. A cell transformed by a plasmid is called a "transformant".

Phage or Bacteriophage - Bacterial virus many of which consist of DNA sequences encapsidated in a protein envelope or coat ("capsid").

Cloning Vehicle - A plasmid, phage DNA or other DNA sequence which is able to replicate in a host cell, characterized by one or a small number of endonuclease recognition sites at which such DNA sequences may be cut in a determinable fashion without attendant loss of an essential biological function of the DNA, e.g., replication, production of coat proteins or loss of promoter or binding sites, and which contain a marker suitable for use in the identification of transformed cells, e.g., tetracycline resistance or ampicillin resistance. A cloning vehicle is often called a vector.

Cloning - The process of obtaining a population of organisms or DNA sequences derived from one such organism or sequence by asexual reproduction.

Recombinant DNA Molecule or Hybrid DNA - A molecule consisting of segments of DNA from different genomes which have been joined end-to-end outside of living cells and able to be maintained in living cells.

Expression Control Sequence - A sequence of nucleotides that controls and regulates expression of genes when operatively linked to those genes. They include the lac system, the trp system, the TAC system, the TRC system, major operator and promoter regions of phage λ, the control region of fd coat protein the early and late promoters of SV40, promoters derived from polyoma virus adenovirus and simian virus, the promoter for 3-phosphoglycerate kinase or other glycolytic enzymes, the promoters of yeast acid phosphatase eg., Pho5, the promoters of the yeast α-mating type factors, and other sequences known to control the expression of genes of prokaryotic or eukaryotic cells and their viruses or combinations thereof.

Stage-Specific Late Schizont-Merozoite P.falciparum Antigen - A polypeptide that is: (1) specific to the late schizont-merozoite development stage of P.falciparum, (2) specifically recognized by a monoclonal antibody specific to a malarial polypeptide that is protective in monkeys, and (3) able to raise antibodies that immunoprecipitate at least one of the malarial polypeptides that are protective in monkeys.

We now describe one embodiment of a process for selecting DNA sequences coding for late schizontmerozoite stage specific antigens of the human malarial parasite P falciparum and the expression of those sequences in hosts transformed with them.

DETERMINATION THAT CERTAIN POLYPEPTIDES ARE SPECIFIC TO THE LATE SCHIZONT- MEROZOITE DEVELOPMENT STAGE OF P. FALCIPARUM

We infected human red blood cells in vitro with the SGE1 isolate of P.falciparum [L. Rodriguez da Silva et al., Bull. WHO, 61, pp. 105-112 (1983)]. We then cultured these cells in vitro according to the method of W. Trager and J.B. Jensen, Science, 193, pp. 673-75 (1976). We achieved synchronization of the cultures as described by Rodriguez da Silva et al., supra , and sequentially labelled the cultures in vitro with pulses of ^35S-methionine (50 μ Ci/ml) in methionine-free MEM supplemented with 10% normal human serum.

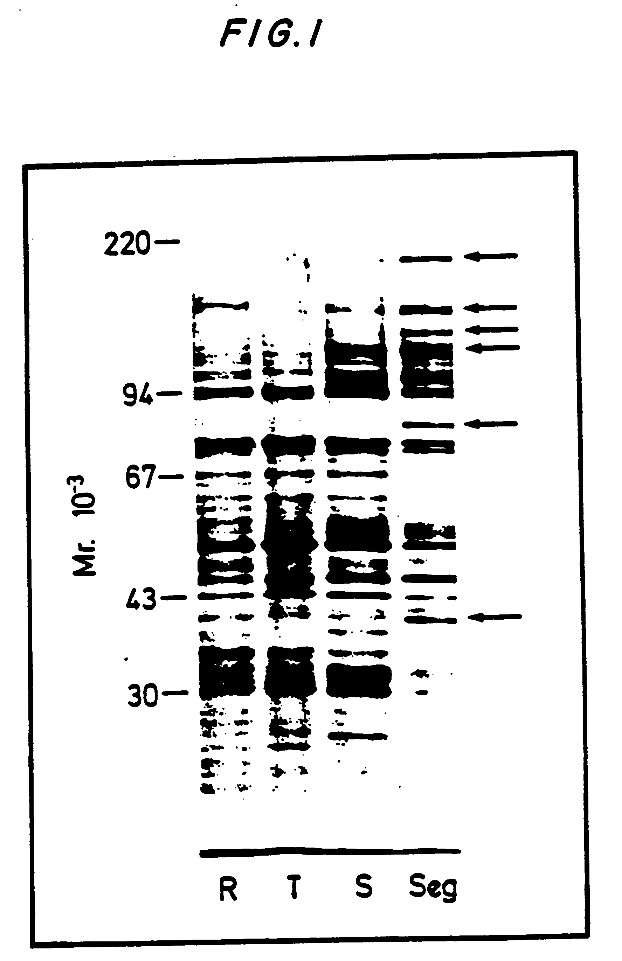

Following lysis with 10% NP40 and centrifugation, we analyzed the extracts containing 30,000 TCA precipitable cpm on acrylamide-SDS gels under reducing conditions [U.K. Laemmli, Nature, 227, pp. 680-83 (1970)]. We observed that the majority of the proteins synthesized were common to the different developmental stages of P.falciparum (Figure 1). However, we also observed that a smaller number of proteins were only synthesized in mature or segmented schizonts which contain merozoites (Figure 1, arrows). The relative intensity of these bands demonstrated that this minor group of proteins was the major constituent of late schizonts and merozoites.

Because these polypeptides were specific to late schizonts and merozoites and because such stage-specific polypeptides have been shown to confer protection in monkeys [Perrin et al., supra ], we isolated poly(A)+ RNA from infected erythrocytes as a means of preparing cDNA and finally cloning and expressing those schizont-merozoite stage-specific polypeptides.

## PREPARATION OF P FALCIPARUM POLY(A)+ RNA

We infected human red blood cells with P.falciparum (isolate SGE2, originating from Zaire) and grew asynchronous cultures to a parasitemia of 15%. We then prepared total RNA from the cultures by homogenization of the infected cells in a solution of 6 M guanidine isothiocyanate, 0.1 M 2-mercaptoethanol, 25 mM sodium citrate (pH 7.0) and 0.5% N-lauroyl-sarcosine and centrifugation of the RNA through 5.7 M cesium chloride [J.M. Chirgwin et al., Biochemistry, 18, pp. 5294-99)]. We then purified the poly(A)+ RNA by affinity chromatography on oligo dT cellulose. We demonstrated that this poly(A)+ RNA was active in cell free protein synthesis.

## CONSTRUCTION OF A cDNA LIBRARY CONTAINING MALARIAL cDNAs AND SCREENING THAT LIBRARY TO SELECT LATE SCHIZONT-MEROZOITE STAGE SPECIFIC RELATED CLONES

We constructed a library of cDNA clones from the above-described polylA)+ RNA by synthesizing cDNA, tailing it with oligo dC and cloning it into pBR322 which had been cleaved with PstI and tailed with oligo dG, substantially as described by C.T. Wake et al., "Isolation Of cDNA Clones Encoding HLA-DR Chains", Proc. Natl. Acad. Sci. USA, 79. pp. 6979-83 (1982).

We then transformed E.coli HB101 with our cDNAs and grew up a library of about 9000 cDNA clones and replicated them onto nitrocellulose filters [D. Hanahan and M. Meselson, Gene, 10, pp. 63-67 (1980)].

In order to screen this library to select late-schizont-merozoite stage specific related clones, we prepared total RNA from RNA preparations enriched for the ring stage and total RNA enriched for the mature schizont-merozoites stage, respectively, using a modification of the procedure described by L. Rodriguez da Silva et al., supra.

We cultivated parasitized red blood cells from the P.falciparum isolate SGE2 (originating from Zaire) in 10 cm diameter Falcon petri dishes at a haematocrit of 6% using 11 ml of culture medium [L. Rodriguez da Silva et al., supra ; A-A. Holder and R.R. Freeman, Nature, 294, pp. 361-64 (1981)]. We synchronized the cells from 25 petri dishes at 22% parasitemia by two treatments of 5% mannitol at 7 h intervals. Twenty-two hours later these cultures had a parasitemia of 9% and contained more than 80% schizonts.

We purified the schizonts by flotation on physiogel and after washing them in culture medium, we mixed them at a ratio of 5:1 with uninfected red blood cells. We limited the re-invasion period to 6 h and then lysed the remaining mature forms by mannitol treatment. We then dispersed the cells into 10 petri dishes. We collected cells from 5 petri dishes after an additional culture period of 6 h (ring form preparation) and the cells from the remaining 5 dishes after an additional culture period of 33 h (mature segmented schizonts). These cultures had a parasitemia of 12% and had respectively more than 97% ring forms (few young trophozoites) and over 95% segmented schizonts (few young schizonts and ring forms), respectively.

From these enriched RNA preparations, we prepared single-stranded 32P-cDNA probes by conventional methodology. We then screened our cDNA library by colony hybridization on the nitrocellulose filters with the single-stranded cDNA probes [M. Grunstein and D. Hogness, Proc. Natl. Acad. Sci. USA , 72, pp. 396-65 (1975)].

We prepared duplicate nitrocellulose filters containing our library of clones for screening with our labelled probes. We prehybridized the filters for 2 h at 65 ° C in 2 x SSC (1 x SSC = 0.15 M NaCl, 0.015 M Na citrate), 1 x Denhardt's with 50 μg/ml of denatured salmon sperm DNA and 1 μg/ml poly A. We then added the labelled probes to a final concentration of 2-4 x 10 cpm/ml and hybridized the filters for 16 h. We then washed the filters 5x for 30 min. in 2 x SSC, 0.1% SDS at 65 ° C and autoradiographed them for about 16 h.

Although most of the colonies hybridized with both cDNA probes, about 150 colonies out of the 9000 colonies screened, preferentially or exclusively hybridized with the late schizont-merozoite enriched cDNA probe. We designated these colonies E.coli HB101 (pBR322(Pst)/X), wherein X represents the position of the particular clone on the filter. For example, one such clone is E.coli HB101 (pBR322(Pst)/ D4). We designated the recombinant DNA molecule that characterized each clone pBR322(Pst)/X and the inserted DNA sequence: X. Again, "X" refers to the position of the particular clone, in the example above: D4.

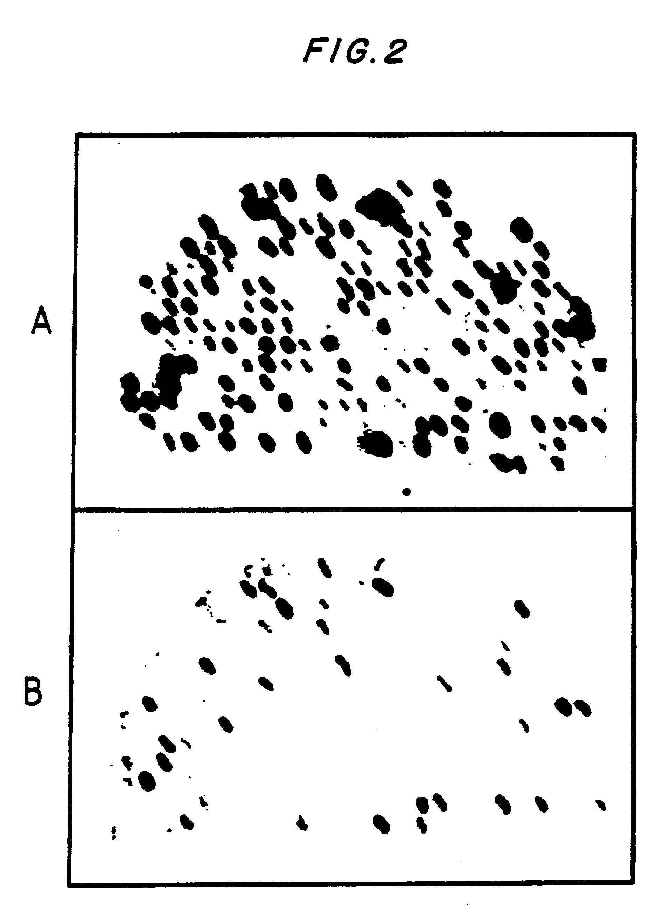

We picked those clones showing a stronger signal with the schizont-merozoite enriched probe onto a gridded filter and replicated the filter as before. We then rehybridized the selected cDNA clones with the two probes under the same conditions described above. The results of this second screening are displayed in Figure 2--(A) representing the screening with the late schizont-merozoite enriched probe and (B) representing the screening with the ring enriched probe.

To confirm that the colonies selected after our second hybridization corresponded to DNA sequences which are activated in a stage-specific manner in the course of P.falciparum development, we prepared RNA (blots) from both the ring and late schizont-merozoite developmental stages, as described above, and hybridized them with $^{32}$P-labelled plasmid DNA from some of our selected clones.

For hybridization we took samples of total RNA (4 μg per track) denatured them and electrophoresed them on a 0.8% agarose gel [G. K. McMaster and G. G. Carmichael, Proc. Natl. Acad. Sci. USA, 74, pp. 4835-38 (1977)]. Ethidium bromide staining demonstrated that there were equal amounts of RNA in both the merozoite (M) and ring (R) tracks. We then transferred the RNA to DBM paper, and conducted our hybridizations and washing substantially as described by G.M. Wahl et al., Proc. Natl. Acad. Sci. USA, 76, pp. 3683-87 (1979). For our hybridization probe, we nick-translated plasmid DNAs from some of our individual late schizont-merozoite stage specific clones [P.W.J. Rigby et al., J. Mol. Biol., 113, pp. 237-51 (1977)]. We used these probes for hybridizations at a concentration of 5 x 10$^5$ cpm ml.

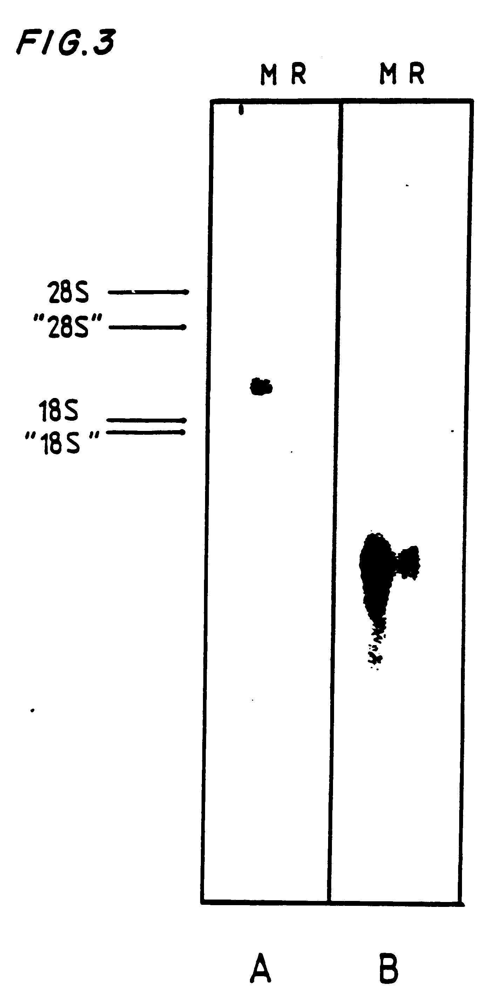

Figure 3 displays the results of two such Northern blot hybridizations: (A) cDNA clone D4; (B) cDNA clone X11; M-late schizont-merozoite RNA blot, R-ring RNA blot. These Northern blots show that the mRNAs have a different size and abundance. They also suggest that activation of new genes at the transcriptional level takes place in this particular parasite developmental pathway. They also demonstrate that the two clones are related to different malarial genes.

## SELECTION OF VARIOUS FAMILIES OF LATE SCHIZONT-MEROZOITE STAGE-SPECIFIC CLONES

To divide our late schizont-merozoite stage specific clones into families of related clones, we used the inserts of some clones as probes to identify related sequences in other clones by hybridization.

We cut out inserts from our individual clones with Pst I, purified them on 1% agarose gel, eluted them using DEAE-cellulose paper [G. Dretzen et al., Anal. Biochem., 112, pp. 295-98 (1981)] and nick translated them [P.W.J. Rigby et al., supra ]. We then used these labelled inserts as hybridization probes under the same conditions as described above for the hybridizations with the $^{32}$P-cDNA merozoite-enriched and ring-enriched probes.

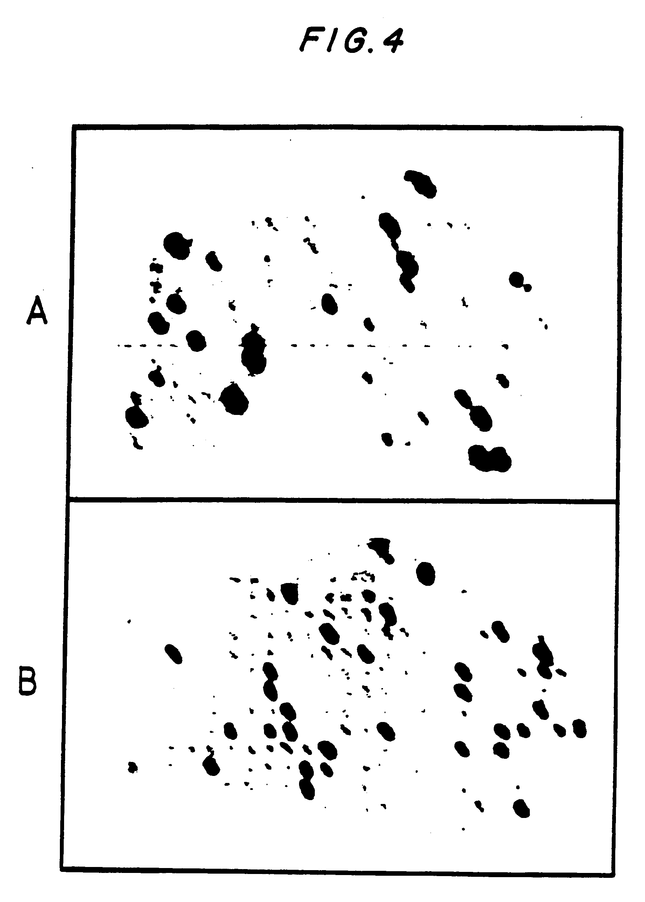

Figure 4 displays an autoradiograph showing the results of this "relatedness" hybridization using replicas of the selected late schizont-merozoite cDNA clones selected previously (Figure 2), and the Pst I-cleaved inserts from clone D4 (A) and V14 (B).

Similar "relatedness" hybridizations permitted us to divide 133 of our late schizont-merozoite stage specific clones into twelve non-overlapping families of clones.* These families and the number of members in each are depicted in Table I. They are designated by the clone that selected them by hybridization.

* The remaining 17 clones, and perhaps others selected as described above, do not belong to any of the twelve families. They may, however, represent other late schizont-merozoite stage specofic families.

## Table I

| Family | Members |
|--------|---------|
| V14 | 31 |
| G2 | 23 |
| R14 | 20 |
| D4 | 19 |
| X11 | 18 |
| M2 | 7 |
| I5 | 4 |
| M7 | 3 |
| T4 | 3 |
| D8 | 2 |
| T4 | 2 |
| E8 | 1 |

On the basis of the number of clones in each family, we have concluded that these 12 families include those DNA sequences that code for the abundant and moderately abundant proteins expressed specifically at the late schizont-merozoite development stage of P.falciparum (see, e.g., Figure 1). On the basis of our Northern RNA blots described previously, we have demonstrated that these twelve families are related to different malarial genes. Therefore, we have concluded that during the development cycle of P.falciparum, maturation of the parasite from the ring stage to the merozoite stage is associated with the selective activation of a relatively small set of DNA sequences. Moreover, these DNA sequences display various degrees of differential activation. Such selective gene activation has also been reported in the development of Drosophilia [E.A. Fyrdeng et al., Cell, 33, pp. 115-123 (1983)], Bombyx Mori [T.H. Eickbush and F.C. Kafatos, Cell, 29, pp. 633-43 (1982)], and Dictostelium [M.C. Mehydy et al., Cell, 32, pp. 763-71 (1983)].

It should be understood that similar hybridizations using similar conditions to those described above and using probes selected from our families of clones may be employed to select other members of these families from collections of DNA sequences from natural, synthetic, semisynthetic or other sources including genomic banks from P.falciparum.

It should also be understood that similar techniques to those described above may be employed to select families of DNA sequences encoding stage-specific late schizont-merozoite antigens from other plasmodium species, such as P. vivax, P. malariae and P. ovale.

## PRODUCTION OF STAGE-SPECIFIC LATE SCHIZONT-MEROZOITE ANTIGENS

To produce the stage-specific late schizontmerozoite antigens encoded by our various families of DNA sequences, we prepared another library of cDNA clones. We prepared this library substantially as described above from poly(A)$^+$ RNA except that we used expression vector pPL3IA-Pst (a gift of H. Kupper) and E.coli MCI06I (pCl857) to clone our cDNAs.

Expression vector pPL3IA-Pst is a derivative of pPLc24 [E. Remaut et al., Gene, 15, pp. 81-93 (1981)]. It is characterized by a PstI restriction endonuclease recognition site downstream of the coding sequence for the MS2 portion of the vector. It is also characterized by a modified ampicillin resistance gene. This modified gene, derived from pUC8 [see, e.g., U. Ruther et al., Mol. Gen. Genet., 178, pp. 475-77 (1980)], does not include a PstI site. Accordingly, the vector has a unique PstI site downstream of the promoter and MS2 coding region in which to insert a cDNA sequence for expression under control of the $P_L$ promoter of the vector.

Our library in this expression vector consisted of about 21,000 clones. We first screened the library with a mixed hybridization probe consisting of probes from each of our previously described 12 families of clones to select those clones related to stage-specific late schizont-merozoite antigens. The hybridization conditions were substantially the same as those described previously for selection of our families of clones. In this manner we selected about 500 clones. We then divided these clones into their respective families by screening them with individual probes from our previously described families. Again the hybridization conditions were substantially the same as those described previously for selection of our families of clones. We isolated representatives of all 12 families from our library.

We randomly selected 15 clones from each of four families of clones (R14, G2, V14 and X11) and grew liquid cultures of them overnight at 30°C in L-Broth, supplemented with ampicillin and kanamycin. We then diluted the cultures 1:4 with L-Broth, supplemented as before at 42 ° C, and induced them for 3 h at 42 ° C.

We handled the resulting cultures in two ways. On one aliquot we lysed the cells with lysozyme, freeze-thawed them, centrifuged them and analyzed the supernatant in a conventional ELISA assay. In this assay we added our cell extract to a support-bound pool of monoclonal antibodies specific to the 2OOK, 82K and 41 K malarial antigens that were protective in monkeys [Perrin et al., supra ]. We then added enzyme-linked rabbit anti-mouse immunoglobulin that had been affinity purified. We detected the positive extracts by colormetric reaction. The results of our assays are displayed in Table II.

On a second aliquot of our cultures, we centrifuged the cells, suspended the pellet in Laemli buffer, boiled the solution, and loaded it onto a polyacryamide-SDS gel under reducing conditions [Laemmli, supra ]. For a control we used a culture prepared from a clone without a malarial cDNA insert. We analyzed our gels by looking for additional bands as compared to the control. The results of our gel assays are also displayed in Table II.

## TABLE II

| Family | | Members That Were Positive* | | |
|---|---|---|---|---|
| | Additional Band** | ELISA*** | | |
| | | 1 | 2 | 3 |
| V14 | C26 | -- | -- | -- |
| | F30 | -- | -- | -- |
| | K24 | -- | -- | K24 |
| | 027 | 027 | -- | 027 |
| X11 | K4 | -- | -- | -- |
| | 011 | 011 | 011 | 011 |
| | X12 | -- | -- | -- |
| R14 | B8 | -- | -- | -- |
| | D12 | -- | D12 | D12 |
| | -- | D15 | -- | -- |
| | -- | J18 | J18 | -- |
| G2 | -- | C11 | C11 | C11 |

Our other families of clones may be analyzed similarly. Western blots of each antigen produced by our clones can also be analyzed using individual monoclonal antibodies that are specific

---

* Because there are two possible orientations and three possible reading frames for each cDNA in our expression vector, there is a 1:6 probability that any cDNA clone will express the correct malarial polypeptide. We chose 15 clones from each family in order to increase the probability of at least one expression.

** These were clones having additional bands that we could observe on the gel. Other clones may have had additional bands that were obscured by other gel bands.

*** Some of the antigens produced by our clones may not be specific to the three types of monoclonal antibodies employed in the assay. Instead, they may be specific to other monoclonal antibodies that are specific to malarial protective antigens.

to malaria protective polypeptides [E.g., L.H. Perrin et al.]. Finally, our malarial antigens may be injected into mice or rabbits and the antibodies raised used to immunoprecipitate malaria protective polypeptides [E.g., L.H Perrin et al.].

We chose clones X11-011 and R14-B8 for further study. We grew liquid cultures of E.coli 537(pPL3lA-Pst(X11-011) and E.coli 537 (pPL3lA-Pst(R14-B8). In L-Broth, supplemented with ampicillin and kanamycin as before, and grew them overnight at 30 ° C. After dilution of the cultures 1:4 with L-Broth, at 43 ° C, we incubated them at 43 ° C for 3 hours. We then prepared extracts by centrifugation, PBS washing, and sonication, and analyzed the resuspended pellets on Western blots with a rabbit antisera that had been raised against whole parasite proteins (Gambia) from which the 4IK protein (L. Perrin et al., supra ) had been removed by affinity chromatography.

The X11-011 extract was characterized by the presence of a protein product (in high yield) that was specifically recognized by the sera. It was also recognized by antisera specific to the 200 kd malarial protein. The R14-B8 extract did not react with the whole parasite antisera described above. However, subsequent immunodiagnostic tests using the blood of infected individuals demonstrated that the R14-B8 antigen existed in infected patients.

We also prepared an extract from our X11-011 culture by suspending the PBS washed pellet in 6M urea and recentrifuging the mixture. The supernatant was then used to inoculate rabbits and to raise antisera. This antisera immuno-precipitates (weakly) the in vivo labelled merozoite parasite 200 kd protein.

Partial DNA sequencing of clone X11-011 revealed, inter alia, repeats of 5 amino acids. However, the sequence did not overlap with any of the sequence determined for clone 31-1 or FCC-1, infra. However, it may still be a part of the same gene or a related one.

We have also obtained partial DNA sequences from clone family D4. These sequences are characterized by repeats of 4 amino acids. However, they also do not overlap with the sequences of X11-011, 31-1 or FCC-1.

PRODUCTION OF OTHER STAGE-SPECIFIC LATE SCHIZONT-MEROZOITE ANTIGENS

In another approach to the production of stage specific late schizont-merozoite antigens of P.falciparum, we prepared malarial cDNA using SI from poly(A)+ RNA, prepared substantially as described previously. We then used this cDNA to prepare a cDNA library in expression vector pPL3IA-Pst using the PstI restriction endonuclease site and dG/dC tailing, substantially as described above.

We inserted our dC-tailed cDNAs into the dG-tailed, PstI linearized vector and transformed competent E.coli C600 (pCI857) (hereinafter "E.coli 537"). We then placed these cells on L Broth plates, supplemented with 40 μg ml ampicillin and 50 μg ml kanamycin, and grew them at 30 ° C overnight and then at 42 ° C for 5 hours. The higher temperature induces the cells of the culture to express the inserted DNA sequence as part of an MS2 fusion protein. See e.g. Kupper et al., supra .

We lysed our colonies in a buffered salt solution containing 5% SDS. We then baked the filters at 65 ° C for 30 min and fixed the proteins on the filters using an electroblot machine. We then screened the proteins fixed from our colonies with a rabbit antisera that had been raised against whole parasite proteins (Gambia) from which the 41 K protein (L. Perrin et al., supra ) had been removed by affinity chromatography. We detected proteins that bound to the antisera with $^{125}$I-staph A protein. In this way, we picked about 40 positive colonies. We transferred these colonies to nitrocellulose filters and screened them as before. We isolated one positive colony: E.coli C600 (pCI857) (pPL3IA-Pst (Pst) 1) (hereinafter "E.coli C600 (p31-19" or "E.coli 537 (p31-1)").

We prepared a liquid culture of E.coli 537 (p31-1) in L-Broth, supplemented with ampicillin and kanamycin as before, and grew it overnight at 30 ° C. After dilution of the culture 1:4 with L-Broth at 43 ° C, we incubated the culture at 43 ° C for 3 h. We then prepared an extract by centrifugation, PBS washing, and sonication, and analyzed the resuspended pellet on a Western blot with the above-described rabbit antiserum. This analysis showed that an about a 30K protein (14K MS2 and 16K malaria antigen) in the extract was specifically recognized by the sera. This protein accounts for 2-3% of the total cellular proteins

We also prepared an extract from a similarly induced culture of E.coli 537 (p31-1) by suspending the PBS washed pellet in 6 M Urea and recentrifuging the mixture. The supernatant then was used to innoculate rabbits and to raise antisera therein. This antisera immunoprecipitated in vivo labelled merozoite parasite proteins. A SDS gel subsequently demonstrated that the precipitated proteins from a schizont preparation were the 200K malarial protective protein and related products (Perrin et al., supra ). In a purified merozoite preparation, the antisera recognized an 83 kd polypeptide that resides on the surface of merozoites. Therefore, our antigen is conserved during maturation of merozoites. Accordingly, the antigen produced by p31-1 is related to a known malarial pro tective antigen. Furthermore, the rabbit antiserum raised against our 31-1 protein reacted in a conventional immunofluorescence assay [P. O'Neill and G. D. Johnson, J. Clin. Pathol., 23, pp. 185-93 (1970)] with P. falciparum asexual blood stages grown in vitro from isolates from a variety of geographical locations, e.g., SGE 2 (Zaire), FUP (Palo Alto), FCR 3 clone A2, FCC 2 (China) and M 25 (Honduras). The antisera reacted with internal structures of fixed schizonts and with the surface of both fixed and unfixed merozoites.

Immunological characterization of the 31-1 protein has identified that protein as a part of the 200 kd protein (Perrin et al., supra ). We have also shown that the antigenic sites encoded by the 31-1 protein are conserved in the 83K merozoite surface antigen that results from the in vivo processing of the 200 kd schizont protein to that merozoite surface protein.

We have also determined the nucleotide sequence of the DNA insert of p31-1 and derived an amino acid sequence from it. For sequencing we restricted p31-1 with Bam HI and Bgl II and employed the Maxam-Gilbert sequencing method.

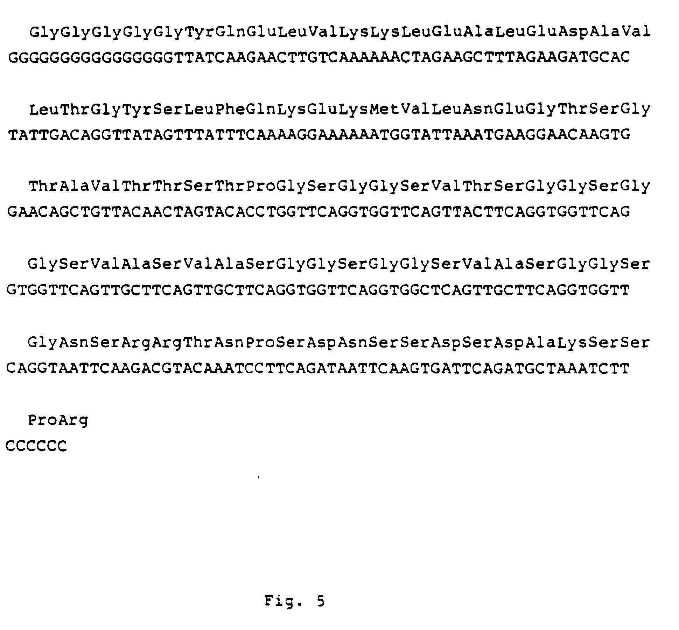

The nucleotide sequence of the DNA insert of p31-1, and the amino acid sequence derived therefrom, is depicted in Figure 5. As depicted in Figure 5, the amino acid sequence derived from the DNA insert of p31-1 is divided into three areas: 43 amino acids of unique sequence, 30 amino acids of tripeptide repeats (ser-gly-gly or ser-val-ala) and a following 20 amino acids of unique sequence. These sequences are not related to other published malarial sequences.

We also used the Bam HI-Bgl II fragment of p31-1, after purification on a gel and nick translation, as a hybridization probe to screen the above-described 150 positive clones containing the twelve families of DNA sequences encoding the stage specific late schizont-merozoite antigens of P.falciparum. We performed the hybridization in 2 x SSC at 65 ° C and used the same conditions to wash the filters. However, we detected no positives. Accordingly, the DNA sequence of clone 31-1, although the clone itself is among the families of stage-specific late schizont-merozoite antigens of P.falciparum, is most likely from a different part of the particular gene that characterizes 31-1's family than any of the 150 clones. Therefore, it does not hybridize to any of those clones.

We also used the Bam HI(Hind III) malarial insert of p31-1, after purification on a gel and nick translation, as a hybridization probe to screen a genomic malarial library prepared in cloning vector pUC9 and E.coli JM103 to select related DNA sequences. We selected several sequences, the largest being designated FCC-1.

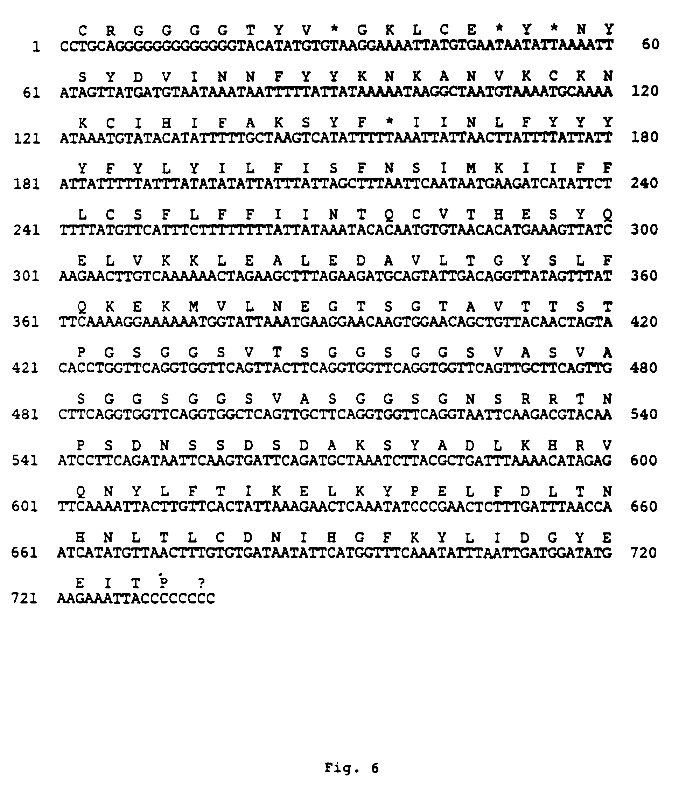

The nucleotide sequence of FCC-1, and its derived amino acid sequence is depicted in Figure 6. As shown in Figure 6, FCC-1 has extended the 31-1 malarial DNA sequence (nucleotides 295-579 in Figure 6) in both the 3′ and 5′ directions. As also shown in Figure 6, the FCC-1 DNA sequence includes a number of stop codons (*) upstream of the start of the 31-1 sequence (e.g., nucleotides 156-158) and a translation start signal (ATG, nucleotides 225-27) in-phase with 31-1. These data suggest that the entire upstream portion of the gene coding for the protein characterized by 31-1 has been cloned. At the 3′ end of FCC-1 the reading frame is still open. Accordingly, we do not believe that we have cloned the complete coding and non-coding regions of this sequence. However, similar hybridizations using 31-1, or more preferably the downstream portions of FCC-1, will enable such cloning.

It should, of course, be understood that because 31-1 expresses a protein product that is useful in raising antibodies against the protective 200 kd malarial protein, it may not be necessary to obtain a full coding sequence for this protein.


USE OF THE DNA SEQUENCES OF THIS INVENTION IN OTHER CONSTRUCTIONS TO PRODUCE STAGE-SPECIFIC LATE SCHIZONT-MEROZOITE ANTIGENS

The DNA sequences of this invention may be used in other expression vector constructions to improve the level of expression and the characteristics of the malarial antigens produced. For example. the DNA sequences may be engineered to produce antigens that are not fused to a portion of MS2 protein or other protein. They may also be engineered to produce fragments of the antigens coded for by them or to produce proteins that include amino acids unrelated to malaria. They may also be used to transform a variety of hosts in order to produce the antigens in those cells. Such hosts include E.coli, Bacillus, Pseudomonas, yeasts, other fungi, Streptomyces, mouse or other animal cells, insect cells, plant cells and human tissue cells. Finally, the amino acid sequences derived from these DNA sequences may be used to prepare synthetic peptides that will be useful as malarial antigens. All that is necessary is that the resulting antigen be a stage-specific late schizont-merozoite antigen. These methods of synthesis and construction are well-known in the art.

For example, we have prepared several synthetic peptides based on the amino acid sequence derived from our clones 31-1 and FCC-1. The first peptide (Peptide A) consisted of 43 amino acid residues from the amino terminal end of the polypeptide coded for by those clones: SYQELVKKLEALEDAVLT GYSLFQKEKM-VLNEGTSGTAVTTST (see Figure 6, Nucleotides 293-422 and Figure 5). It was selected from the "unique" region of the clones. The second peptide (Peptide B) consisted of the 23 carboxy terminal amino acids of Peptide A. The third peptide (Peptide C) consisted of 34 amino acid residues from the repeating region of clones 31-1 and FCC-1: SGGSVTSGGSGGSVASVASGGSGGSVASGGSGNS (see Figure 6, Nucleotides 429-530 and Figure 5).

Each of these peptides raised antibodies in rabbits that reacted in Western blots, immunofluorescence stainings and immunoprecipitation with the 200 kd schizont protein and its processed 83 kd merozoite surface protein. Significantly, these antibodies also reacted with the internal structures of schizonts and with the surface of merozoites from a variety of isolates of P.falciparum in indirect immunofluorescence assays. Again, these results demonstrate that our antigen is conserved during the maturation of merozoites and is likely to be of value as a broad spectrum vaccine against malaria infections.

We employed Peptide A after coupling to Tetanus Toxoid by the conventional glutaraldehyde method and admixture with Freund's complete adjuvant to vaccinate monkeys. After challenge with malarial parasites, the vaccinated monkeys were partially protected (>90%) by the peptide. Accordingly, in vivo our antigens are protective.

## USE OF THE ANTIGENS AND DNA SEQUENCES OF THIS INVENTION IN DIAGNOSTIC METHODS AND MEANS FOR MALARIAL INFECTIONS

The antigens, their antibodies, and the DNA sequences of this invention may be used in methods and means designed to detect the presence of malarial infections in various blood samples.

For example, the antigens produced in this invention, or antibodies raised against them, may be employed in a variety of diagnostic means and methods, e.g., those based on radioimmunoassay, ELISA or non-radioimmunoassay. The DNA sequences of this invention could be employed in diagnostic means and methods based upon Southern hybridization.

For example, in one type of radioimmunoassay, antibodies raised in a laboratory animal to at least one of the antigens of this invention is attached to a solid phase, for example, the inside of a test tube. Antigen is then added to the tube so as to bind to the antibody. A sample of patient's serum, together with a known amount of radioactively labelled (e.g., $125_I$) antibody raised against the antigen of this invention, is then added to the tube. Any stage-specific late schizont-merozoite antibody present in the patient's serum will compete with the labelled antibody for the free antigenic sites on the antigen-antibody complex bond on the support. After the serum has been allowed to react, the tube is washed and the amount of radioactivity bond in the tube is measured. A positive result, i.e., that the patient's serum contains the malaria antibody is indicated by a low level of radioactivity.

In one type of ELISA assay, a microtiter plate is coated with an antigen of this invention and a sample of a patient's serum is added. After a period of incubation permitting interaction of any stage-specific late schizont-merozoite antibody in the serum with the antigen, the plate is washed and an anti-human antibody linked to an enzyme label is added. After incubation, the plate is rewashed. Thereafter, an enzyme substrate is added and the absorbance of the final plate measured. A large change in absorbance indicates a positive result.

## USE OF THE ANTIGENS OF THIS INVENTION IN METHODS AND COMPOSITIONS FOR PROTECTING AGAINST MALARIAL INFECTIONS

The antigens of this invention may also be employed in a variety of pharmaceutically acceptable compositions to protect against malarial infections. Such compositions may include various pharmaceutically acceptable carriers or adjuvants. They may also include compounds that enhance the immune response. These may include various lymphokines and interferons. Preferably, such vaccines include a mixture of antigens selected from two or more of our twelve families. More preferably, the vaccines of this invention are multivalent, i-e., include antigens from one or more of the other plasmodia that infect humans. These mixtures may be the most effective in protecting humans against malarial infections.

These compositions may be used in a variety of pharmaceutically acceptable dosage forms and means of treatment well known in the vaccine art.

Microorganisms of this invention are exemplified by cultures deposited in the Deutsche Sammlung von Mikroorganismen, Gottingen, West Germany,

on February 13, 1984 and identified as PF-1 to 14:

```
PF-1:      E.coli HB101(pBR322(Pst)/I4)
PF-2:      E.coli HB101(pBR322(Pst)/G2)
PF-3:      E.coli HB101(pBR322(Pst)/V4)
PF-4:      E.coli HB101(pBR322(Pst)/D8)
PF-5:      E.coli HB101(pBR322(Pst)/X11)
PF-6:      E.coli HB101(pBR322(Pst)/M2)
PF-7:      E.coli HB101(pBR322(Pst)/M7)
PF-8:      E.coli HB101(pBR322(Pst)/E8)
PF-9:      E.coli HB101(pBR322(Pst)/R14)
PF-10:     E.coli HB101(pBR322(Pst)/D4)


PF-11:     E. coli HB101 (pBR322(Pst)/V14)
PF-12:     E. coli HB101 (pBR322(Pst)/T5)
PF-13:     E. coli 537 (pPL31A-Pst(Pst)/X11-011)
PF-14:     E. coli K12λ (pPL31A-Pst(Pst)/1)
```

They have been assigned accession numbers DSM 2878 through 2891.

Microorganisms and DNA sequences of this invention are also exemplified by a culture deposited in the American Type Culture Collection on February 19, 1985 and identified as PF-15:

PF-15: E.coli JM103(pFCC-1)

It has been assigned accession number 53035.

## Claims

1. A DNA sequence coding on expression for a stage-specific late schizont-merozoite malarial antigen inducing immunity to malaria in primates, which DNA sequence is selected from the group of DNA sequences which hybridize under salt and temperature conditions equivalent to 2 x SSC and 65 °C to a DNA sequence enriched in late schizont-merozoite DNA but do not hybridize under those conditions to a DNA sequence enriched in ring DNA, said DNA enrichments being afforded by a method comprising the steps of culturing red blood cells parasitized by a species of plasmodium, until they contained more than 80 % schizonts, purifying the schizonts from the culture, infecting uninfected red blood cells with the purified schizonts for a period of about six hours, lysing the remaining mature forms, dispersing and culturing the cells, collecting RNA enriched in ring RNA from a first aliquot of the cells after about 6 h, collecting RNA enriched in late schizont-merozoite RNA from a second aliquot of the cells after about 33 h, and reverse transcribing said RNAs into cDNA; DNA sequences which hybridize under salt and temperature conditions equivalent to 2 x SSC and 65 °C to said DNA sequences; and DNA sequences that code on expression for a polypeptide coded on expression by any of the foregoing DNA sequence.

2. The DNA sequence according to claim 1, wherein said malaria antigen is selected from the group of antigen of **p. falciparum, p. vivax, P. malariae and P. ovale.**

3. A DNA sequence selected from the group consisting of the DNA inserts of pBR322 (Pst)V14 identified as Accession No. DSM 2888, DNA sequences which hybridize to said DNA insert under salt and temperature conditions equivalent to 2 x SSC and 65 ° C, and DNA sequences that code on expression for a polypeptide coded for on expression by any of the foregoing DNA inserts and sequences.

15

4. A DNA sequence selected from the group consisting of the DNA inserts of pBR322 (Pst)/G2 identified as Accession No. DSM 2879, DNA sequences which hybridize to said DNA insert under salt and temperature conditions equivalent to 2 x SSC and 65 ° C, and DNA sequences that code on expression for a polypeptide coded for on expression by any of the foregoing DNA inserts and sequences.

5. A DNA sequence selected from the group consisting of the DNA inserts of pBR322 (Pst)/R14 identified as Accession No. DSM 2886, DNA sequences which hybridize to said DNA insert under salt and temperature conditions equivalent to 2 x SSC and 65 ° C, and DNA sequences that code on expression for a polypeptide coded for on expression by any of the foregoing DNA inserts and sequences.

6. A DNA sequence selected from the group consisting of the DNA inserts of pBR322 (Pst)/D4 identified as Accession No. DSM 2887, DNA sequences which hybridize to said DNA insert under salt and temperature conditions equivalent to 2 x SSC and 65 ° C, and DNA sequences that code on expression for a polypeptide coded for on expression by any of the foregoing DNA inserts and sequences.

7. A DNA sequence selected from the group consisting of the DNA inserts of pBR322 (Pst)/XII identified as Accession No. DSM 2882, DNA sequences which hybridize to said DNA insert under salt and temperature conditions equivalent to 2 x SSC and 65 ° C, and DNA sequences that code on expression for a polypeptide coded for on expression by any of the foregoing DNA inserts and sequences.

8. A DNA sequence selected from the group consisting of the DNA inserts of pBR322 (Pst)/M2 identified as Accession No. DSM 2883, DNA sequences which hybridize to said DNA insert under salt and temperature conditions equivalent to 2 x SSC and 65 ° C, and DNA sequences that code on expression for a polypeptide coded for on expression by any of the foregoing DNA inserts and sequences.

9. A DNA sequence selected from the group consisting of the DNA inserts of pBR322 (Pst)/T5 identified as Accession No. DSM 2889, DNA sequences which hybridize to said DNA insert under salt and temperature conditions equivalent to 2 x SSC and 65 ° C, and DNA sequences that code on expression for a polypeptide coded for on expression by any of the foregoing DNA inserts and sequences.

10. A DNA sequence selected from the group consisting of the DNA inserts of pBR322 (Pst) M7 identified as Accession No. DSM 2884, DNA sequences which hybridize to said DNA insert under salt and temperature conditions equivalent to 2 x SSC and 65 ° C, and DNA sequences that code on expression for a polypeptide coded for on expression by any of the foregoing DNA inserts and sequences.

11. A DNA sequence selected from the group consisting of the DNA inserts of pBR322 (Pst)/V4 identified as Accession No. DSM 2880, DNA sequences which hybridize to said DNA insert under salt and temperature conditions equivalent to 2 x SSC and 65 ° C, and DNA sequences that code on expression for a polypeptide coded for on expression by any of the foregoing DNA inserts and sequences.

12. A DNA sequence selected from the group consisting of the DNA inserts of pBR322 (Pst) D8 identified as Accession No. DSM 2881, DNA sequences which hybridize to said DNA insert under salt and temperature conditions equivalent to 2 x SSC and 65 ° C, and DNA sequences that code on expression for a polypeptide coded for on expression by any of the foregoing DNA inserts and sequences.

13. A DNA sequence selected from the group consisting of the DNA inserts of pBR322 (Pst) 14 identified as Accession No. DSM 2878, DNA sequences which hybridize to said DNA insert under salt and temperature conditions equivalent to 2 x SSC and 65 ° C, and DNA sequences that code on expression for a polypeptide coded for on expression by any of the foregoing DNA inserts and sequences.

14. A DNA sequence selected from the group consisting of the DNA inserts of pBR322 (Pst) E8 identified as Accession No DSM 2885, DNA sequences which hybridize to said DNA insert under salt and temperature conditions equivalent to 2 x SSC and 65 ° C, and DNA sequences that code on expression for a polypeptide coded for on expression by any of the foregoing DNA inserts and sequences.

15. A DNA sequence selected from the group consisting of the DNA inserts of PPL3IA-Pst(Pst) 1 identified as Accession No. DSM 2891, PPL3IA-Pst(Pst) XII-011 identified as Accession No. DSM 2890, DNA sequences which hybridize to said DNA inserts under salt and temperature conditions equivalent to 2 x SSC and 65 ° C, and DNA sequences that code on expression for a polypeptide coded for on expression by any of the foregoing DNA inserts and sequences.

16. The DNA sequence according to claim 15, selected from the group consisting of the malarial insert of pFCC-1 as listed in Fig. 6 and identified as Accession No. ATCC 53035, DNA sequences which hybridize to said DNA insert under salt and temperature conditions equivalent to 2 x SSC and 65 ° C, and DNA sequences that code on expression for a polypeptide coded for on expression by any of the foregoing DNA inserts and sequences.

17. A recombinant DNA molecule comprising a DNA sequence selected from the group consisting of the DNA sequences of any one of claims 1 - 16.

18. The recombinant DNA molecule according to claim 17, wherein said molecule further comprises an expression control sequence, said expression control sequence being operatively linked to said DNA sequence of any one of claims 1 - 16.

19. A host transformed with at least one recombinant DNA molecule selected from the group consisting of the recombinant DNA molecules of claim 17 or 18.

20. The host according to claim 19, selected from the group consisting of E.coli, Bacillus, Pseudomonas, yeasts, other fungi, streptomyces, mouse or other animal cells, insect cells, plant cells and human tissue cells.

21. A polypeptide coded for on expression by a DNA sequence selected from the group consisting of DNA sequences of any one of claims 3 - 16.

22. A polypeptide produced by a method of culturing a host transformed with a recombinant DNA molecule selected from the group consisting of the DNA molecules of claims 3 - 16, wherein said molecules further comprise an expression control sequence. said expression control sequence being operatively linked to said molecules.

23. A method for selecting a DNA sequence that codes on expression for a stage specific late schizont-merozoite malarial antigen from a group of DNA sequences comprising the step of determining which of said sequences hybridizes under salt and temperature conditions equivalent to 2 x SSC and 65 ° C to a DNA sequence selected from the group consisting of the DNA sequences of any one of claims 1 - 16.

24. A method for selecting a DNA sequence that codes on expression for a stage specific late schizont-merozoite malarial antigen and that induces immunity to malaria in primates from a group of DNA sequences comprising the step of determining which of said DNA sequences hybridizes under salt and temperature conditions equivalent to 2 x SSC and 65 ° C to a DNA sequence enriched in late schizont-merozoite DNA but does not hybridize under those conditions to a DNA sequence enriched in ring DNA, said DNA enrichments being afforded by a method comprising the steps of culturing red blood cells parasitized by a species of plasmodium, until they contained more than 80 % schizonts, purifying the schizonts from the culture, infecting uninfected red blood cells with the purified schizonts for a period of about six hours, lysing the remaining mature form, dispersing and culturing the cells, collecting RNA enriched in ring RNA form a first aliquot of the cell after about 6 h, collecting RNA enriched in late schizont-merozoite RNA from a second aliquot of the cells after about 33 h, and reverse transcribing said RNAs into cDNA.

25. The method according to claim 23 or 24, wherein said group of DNA sequences is selected from DNA sequences derived from natural, synthetic or semi-synthetic sources.

26. The method according to any one of claims 23 to 25, wherein said malarial antigen is selected from the group consisting of antigens of P.falciparum, P.vivax, P.malariae and P.ovale.

27. A method for producing a stage specific late schizontmerozoite antigen comprising the steps of culturing a host according to claim 19 and collecting the antigen.

28. The method according to claim 27, wherein said host is selected from the group consisting of E.coli, Bacillus, Pseudomonas, yeasts, other fungi, Streptomyces, mouse or other animal cells, insect cells, plant cells and human tissue cells.

29. A pharmaceutically acceptable composition for protecting against malarial infections comprising an amount of at least one polypeptide selected from the group consisting of the polypeptides of claim 21 or 22 effective to raise a protective level of circulating antibodies upon release of merozoites from red blood cells.

30. A composition for diagnosing a malarial infection in a sample of blood serum comprising a composition which contains at least one of the DNA sequences of any one of claims 1 to 16 or at least one of the polypeptides of claim 21 or 22 and/or at least one of an antibody to a polypeptide of claim 21 or 22.

31. A method for diagnosing a malarial infection comprising the step of assaying a sample of blood serum for the presence of a composition which contains the DNA sequences of any one of claims 1 to 16 or at least one of the polypeptides of claim 21 or 22 or at least one of an antibody to a polypeptide of claim 21 or 22.

**Patentansprüche**

1. DNA-Sequenz, welche bei der Expression für ein Malaria-Antigen kodiert, welches spezifisch für ein spätes Schizonten-Merozoiten-Entwicklungsstadium ist, und welches Antigen in Primaten Immunität gegenüber Malaria induziert, und welche DNA-Sequenz aus der Gruppe von DNA-Sequenzen ausgewählt ist, die sich unter Salz- und Temperaturbedingungen, welche 2 x SSC und 65° C äquivalent sind, an eine DNA-Sequenz hybridisieren, die mit einer dem späten Schizonten-Merozoiten-Entwicklungsstadium entsprechenden DNA angereichert ist, die sich aber unter diesen Bedingungen nicht an eine DNA-Sequenz hybridisieren, welche mit einer dem Entwicklungsstadium der Ringformen entsprechenden DNA angereichert ist, wobei diese DNA-Anreicherungen nach einer Methode vorgenommen worden sind, welche die Stufen des Kultivierens von durch eine Plasmodium-Spezies parasitierten Erythrozyten, so lange, bis dieselben mehr als 80 % Schizonten enthielten, des Abtrennens der Schizonten von der Kultur unter Reinigung, des Infizierens nicht-infizierter Erythrozyten mit den gereinigten Schizonten während einer Zeitspanne von etwa sechs Stunden, der Durchführung einer Lyse an den restlichen, reifen Formen, des Dispergierens und Kultivierens der Zellen, des Sammelns von RNA, welche mit dem Entwicklungsstadium der Ringformen entsprechender RNA angereichert ist, aus einem ersten aliquoten Anteil der Zellen nach etwa 6 h, des Sammelns von RNA, welche mit dem späten Schizonten-Merozoiten-Entwicklungsstadium entsprechender RNA angereichert ist, aus einem zweiten aliquoten Anteil der Zellen nach etwa 33 h, und der reversen Transkription dieser RNAs zu cDNAs umfaßte; DNA-Sequenzen, die sich unter Salz- und Temperaturbedingungen, welche 2 x SSC und 65 ° C äquivalent sind, an diese DNA-Sequenzen hybridisieren; und DNA-Sequenzen, welche bei der Expression für ein Polypeptid kodieren, welches seinerseits bei der Expression durch irgendeine der vorstehenden DNA-Sequenzen kodiert wird.

2. DNA-Sequenz nach Anspruch 1, wobei das Malaria-Antigen aus der Gruppe von Antigenen des P. falciparum, P. vivax, P. malariae und P. ovale ausgewählt ist.

3. DNA-Sequenz, welche aus der Gruppe ausgewählt ist, welche aus den als Hinterlegungsnummer DSM 2888 identifizierten DNA-Inserts des pBR322 (Pst)/V14, DNA-Sequenzen, welche sich an dieses DNA-Insert unter Salz- und Temperaturbedingungen hybridisieren, welche 2 x SSC und 65° C äquivalent sind, und DNA-Sequenzen besteht, welche bei der Expression für ein Polypeptid kodieren, welches seinerseits bei, der Expression durch irgendeines der vorstehenden DNA-Inserts und irgendeine der vorstehenden DNA-Sequenzen kodiert wird.

4. DNA-Sequenz, welche aus der Gruppe ausgewählt ist, welche aus den als Hinterlegungsnummer DSM 2879 identifizierten DNA-Inserts des pBR322 (Pst)/G2, DNA-Sequenzen, welche sich an dieses DNA-Insert unter Salz- und Temperaturbedingungen hybridisieren, welche 2 x SSC und 65° C äquivalent sind, und DNA-Sequenzen besteht, welche bei der Expression für ein Polypeptid kodieren, welches seinerseits bei der Expression durch irgendeines der vorstehenden DNA-Inserts und irgendeine der vorstehenden DNA-Sequenzen kodiert wird.

5. DNA-Sequenz, welche aus der Gruppe ausgewählt ist, welche aus den als Hinterlegungsnummer DSM 2886 identifizierten DNA-Inserts des pBR322 (Pst)/R14, DNA-Sequenzen, welche sich an dieses DNA-Insert unter Salz- und Temperaturbedingungen hybridisieren, welche 2 x SSC und 65° C äquivalent sind, und DNA-Sequenzen besteht, welche bei der Expression für ein Polypeptid kodieren, welches seinerseits bei der Expression durch irgendeines der vorstehenden DNA-Inserts und irgendeine der vorstehenden DNA-Sequenzen kodiert wird.

6. DNA-Sequenz, welche aus der Gruppe ausgewählt ist, welche aus den als Hinterlegungsnummer DSM 2887 identifizierten DNA-Inserts des pBr322 (Pst)/D4, DNA-Sequenzen, welche sich an dieses DNA-Insert unter Salz- und Temperaturbedingungen hybridisieren, welche 2 x SSC und 65° C äquivalent sind, und DNA-Sequenzen besteht, welche bei der Expression für ein Polypeptid kodieren, welches seinerseits bei der Expression durch irgendeines der vorstehenden DNA-Inserts und irgendeine der vorstehenden DNA-Sequenzen kodiert wird.

7. DNA-Sequenz, welche aus der Gruppe ausgewählt ist, welche aus den als Hinterlegungsnummer DSM 2882 identifizierten DNA-Inserts des pBR322 (Pst)/X11, DNA-Sequenzen, welche sich an dieses DNA-Insert unter Salz- und Temperaturbedingungen hybridisieren, welche 2 x SSC und 65° C äquivalent sind, und DNA-Sequenzen besteht, welche bei der Expression für ein Polypeptid kodieren, welches seinerseits bei der Expression durch irgendeines der vorstehenden DNA-Inserts und irgendeine der vorstehenden DNA-Sequenzen kodiert wird.

8. DNA-Sequenz, welche aus der Gruppe ausgewählt ist, welche aus den als Hinterlegungsnummer DMS 2883 identifizierten DNA-Inserts des pBR322 (Pst)/M2, DNA-Sequenzen, welche sich an dieses DNA-Insert unter Salz- und Temperaturbedingungen hybridisieren, welche 2 x SSC und 65° C äquivalent sind, und DNA-Sequenzen besteht, welche bei der Expression für ein Polypeptid kodieren, welches seinerseits bei der Expression durch irgendeines der vorstehenden DNA-Inserts und irgendeine der vorstehenden DNA-Sequenzen kodiert wird.

9. DNA-Sequenz, welche aus der Gruppe ausgewählt ist, welche aus den als Hinterlegungsnummer DSM 2889 identifizierten DNA-Inserts des pBR322 (Pst)/T5, DNA-Sequenzen, welche sich an dieses DNA-Insert unter Salz- und Temperaturbedingungen hybridisieren, welche 2 x SSC und 65° C äquivalent sind, und DNA-Sequenzen besteht, welche bei der Expression für ein Polypeptid kodieren, welches seinerseits bei der Expression durch irgendeines der vorstehenden DNA-Inserts und irgendeine der vorstehenden DNA-Sequenzen kodiert wird.

10. DNA-Sequenz, welche aus der Gruppe ausgewählt ist, welche aus den als Hinterlegungsnummer DSM 2884 identifizierten DNA-Inserts des pBR322 (Pst)/M7, DNA-Sequenzen, welche sich an dieses DNA-Insert unter Salz- und Temperaturbedingungen hybridisieren, welche 2 x SSC und 65° C äquivalent sind, und DNA-Sequenzen besteht, welche bei der Expression für ein Polypeptid kodieren, welches seinerseits bei der Expression durch irgendeines der vorstehenden DNA-Inserts und irgendeine der vorstehenden DNA-Sequenzen kodiert wird.

11. DNA-Sequenz, welche aus der Gruppe ausgewählt ist, welche aus den als Hinterlegungsnummer DSM 2880 identifizierten DNA-Inserts des pBR322 (Pst)/V4, DNA-Sequenzen, welche sich an dieses DNA-Insert unter Salz- und Temperaturbedingungen hybridisieren, welche 2 x SSC und 65° C äquivalent sind, und DNA-Sequenzen besteht, welche bei der Expression für ein Polypeptid kodieren, welches seinerseits bei der Expression durch irgendeines der vorstehenden DNA-Inserts und irgendeine der vorstehenden DNA-Sequenzen kodiert wird.

12. DNA-Sequenz, welche aus der Gruppe ausgewählt ist, welche aus den als Hinterlegungsnummer DSM 2881 identifizierten DNA-Inserts des pBR322 (Pst)/D8, DNA-Sequenzen, welche sich an dieses DNA-Insert unter Salz- und Temperaturbedingungen hybridisieren, welche 2 x SSC und 65° C äquivalent sind, und DNA-Sequenzen besteht, welche bei der Expression für ein Polypeptid kodieren, welches seinerseits bei der Expression durch irgendeines der vorstehenden DNA-Inserts und irgendeine der vorstehenden DNA-Sequenzen kodiert wird.

13. DNA-Sequenz, welche aus der Gruppe ausgewählt ist, welche aus den als Hinterlegungsnummer DSM 2878 identifizierten DNA-Inserts des pBR322 (Pst)/I4, DNA-Sequenzen, welche sich an dieses DNA-Insert unter Salz- und Temperaturbedingungen hybridisieren, welche 2 x SSC und 65° C äquivalent sind, und DNA-Sequenzen besteht, welche bei der Expression für ein Polypeptid kodieren, welches seinerseits bei der Expression durch irgendeines der vorstehenden DNA-Inserts und irgendeine der vorstehenden DNA-Sequenzen kodiert wird.

14. DNA-Sequenz, welche aus der Gruppe ausgewählt ist, welche aus den als Hinterlegungsnummer DSM 2885 identifizierten DNA-Inserts des pBR322 (Pst)/E8, DNA-Sequenzen, welche sich an dieses DNA-Insert unter Salz- und Temperaturbedingungen hybridisieren, welche 2 x SSC und 65° C äquivalent sind, und DNA-Sequenzen besteht, welche bei der Expression für ein Polypeptid kodieren, welches seinerseits bei der Expression durch irgendeines der vorstehenden DNA-Inserts und irgendeine der vorstehenden DNA-Sequenzen kodiert wird.

15. DNA-Sequenz, welche aus der Gruppe ausgewählt ist, welche aus den als Hinterlegungsnummer DSM 2891 identifizierten DNA-Inserts des pPL31A-Pst(Pst)/1, den als Hinterlegungsnummer DSM 2890 identifizierten DNA-Inserts des pPL31A-Pst(Pst)/X11-011, DNA-Sequenzen, welche sich an diese DNA-Inserts unter Salz- und Temperaturbedingungen hybridisieren, welche 2 x SSC und 65° C äquivalent sind, und DNA-Sequenzen besteht, welche bei der Expression für ein Polypeptid kodieren, welches seinerseits bei der Expression durch irgendeines der vorstehenden DNA-Inserts und irgendeine der vorstehenden DNA-Sequenzen kodiert wird.

16. DNA-Sequenz nach Anspruch 15, welche aus der Gruppe ausgewählt ist, welche aus dem als Hinterlegungsnummer ATCC 53035 identifizierten Malaria-Insert des pFCC-1, wie dieses Insert in der Fig. 6 angeführt ist, DNA-Sequenzen, welche sich an dieses DNA-Insert unter Salz- und Temperaturbedingungen hybridisieren, welche 2 x SSC und 65° C äquivalent sind, und DNA-Sequenzen besteht, welche bei der Expression für ein Polypeptid kodieren, welches seinerseits bei der Expression durch irgendeines der vorstehenden DNA-Inserts und irgendeine der vorstehenden DNA-Sequenzen kodiert wird.

17. Rekombinations-DNA-Molekül, welches eine DNA-Sequenz enthält, welche aus der Gruppe ausgewählt ist, die aus den DNA-Sequenzen nach einem der Ansprüche 1 bis 16 besteht.

18. Rekombinations-DNA-Molekül nach Anspruch 17, wobei dieses Molekül weiterhin eine die Expression regelnde Sequenz enthält, wobei diese die Expression regelnde Sequenz in Wirkverbindung mit der DNA-Sequenz nach einem der Ansprüche 1 bis 16 steht.

19. Wirt, der mit wenigstens einem Rekombinations-DNA-Molekül transformiert worden ist, welches aus der Gruppe ausgewählt ist, die aus den Rekombinations-DNA-Molekülen der Ansprüche 17 oder 18 besteht.

20. Wirt nach Anspruch 19, der aus der Gruppe ausgewählt ist, die aus E. coli, Bacillus, Pseudomonas, Hefen, anderen Pilzen, Streptomyces, Mäusezellen oder anderen tierischen Zellen, Insektenzellen, Pflanzenzellen und Humangewebezellen besteht.

21. Polypeptid, welches bei der Expression von einer DNA-Sequenz kodiert wird, welche aus der Gruppe ausgewählt ist, die aus DNA-Sequenzen nach einem der Ansprüche 3 bis 16 besteht.

22. Polypeptid, welches nach einem Verfahren erzeugt worden ist, welches das Kultivieren eines mit einem Rekombinations-DNA-Molekül transformierten Wirtes unfaßt, welches Molekül aus der Gruppe ausgewählt ist, welche aus den DNA-Molekülen der Ansprüche 3 bis 16 besteht, wobei diese Moleküle weiterhin eine die Expression regelnde Sequenz enthalten, und wobei diese die Expression regelnde Sequenz in Wirkverbindung mit den Molekülen steht.

23. Verfahren zur Auswahl einer DNA-Sequenz, welche bei der Expression für ein Malaria-Antigen kodiert, welches Antigen spezifisch für ein spätes Schizonten-Merozoiten-Entwicklungsstadium ist, aus einer Gruppe von DNA-Sequenzen, welches Verfahren die Stufe des Feststellens umfaßt, welche dieser DNA-Sequenzen sich unter Salz- und Temperaturbedingungen, welche 2 x SSC und 65° C äquivalent sind, an eine DNA-Sequenz hybridisiert, die aus der Gruppe ausgewählt ist, die aus den DNA-Sequenzen nach einem der Ansprüche 1 bis 16 besteht.

24. Verfahren zur Auswahl einer DNA-Sequenz, welche bei der Expression für ein Malaria-Antigen kodiert, welches Antigen spezifisch für ein spätes Schizonten-Merozoiten-Entwicklungsstadium ist, und welches Antigen in Primaten Immunität gegenüber Malaria induziert, aus einer Gruppe von DNA-Sequenzen, welches Verfahren die Stufe des Feststellens umfaßt, welche dieser DNA-Sequenzen sich unter Salz- und Temperaturbedingungen, welche 2 x SSC und 65° C äquivalent sind, an eine DNA-Sequenz hybridisiert, die mit einer dem späten Schizonten-Merozoiten-Entwicklungsstadium entsprechenden DNA angereichert ist, die sich aber unter diesen Bedingungen nicht an eine DNA-Sequenz hybridisiert, welche mit einer dem Entwicklungsstadium der Ringformen entsprechenden DNA angereichert ist, wobei diese DNA-Anreicherungen nach einer Methode vorgenommen worden sind, welche die Stufen des Kultivierens von durch eine Plasmodium-Spezies parasitierten Erythrozyten, so lange, bis dieselben mehr als 80 % Schizonten enthielten, des Abtrennens der Schizonten von der Kultur unter Reinigung, des Infizierens nicht-infizierter Erythrozyten mit den gereinigten Schizonten während einer Zeitspanne von etwa sechs Stunden, der Durchführung einer Lyse an den restlichen, reifen Formen, des Dispergierens und Kultivierens der Zellen, des Sammelns von RNA, welche mit dem Entwicklungsstadium der Ringformen entsprechender RNA angereichert ist, aus einem ersten aliquoten Anteil der Zellen nach etwa 6 h, des Samnelns von RNA, welche mit dem späten Schizonten-Merozoiten-Entwicklungsstadium entsprechender RNA angereichert ist, aus einem zweiten aliquoten Anteil der Zellen nach etwa 33 h, und der reversen Transkription dieser RNAs zu cDNAs umfaßte.

25. Verfahren nach Anspruch 23 oder 24, wobei diese Gruppe von DNA-Sequenzen aus DNA-Sequenzen ausgewählt wird, welche aus natürlichen, synthetischen oder semi-synthetischen Quellen stammen.

26. Verfahren nach einem der Ansprüche 23 bis 25, wobei das Malaria- Antigen aus der Gruppe ausgewählt ist, welche aus den Antigenen von P. falciparum, P. vivax, P. malariae und P. ovale besteht.

27. Verfahren zur Herstellung eines Antigens, welches spezifisch für ein spätes Schizonten-Merozoiten-Entwicklungsstadium ist, welches Verfahren die Stufen des Kultivierens eines Wirtes nach Anspruch 19 und des Sammelns des Antigens umfaßt.

28. Verfahren nach Anspruch 27, wobei der Wirt aus der Gruppe ausgewählt ist, die aus E. coli, Bacillus, Pseudomonas, Hefen, anderen Pilzen, Streptomyces, Mäusezellen oder anderen tierischen Zellen, Insektenzellen, Pflanzenzellen und Humangewebezellen besteht.

29. Pharmazeutisch annehmbare Zusammensetzung, zum Schutz gegen Malaria-Infektionen, welche eine Menge von wenigstens einem Polypeptid enthält, das aus der Gruppe ausgewählt ist, die aus den Polypeptiden der Ansprüche 21 oder 22 besteht, und welche Zusammensetzung dahingehend wirksam ist, nach Freisetzung von Merozoiten aus Erythrozyten zur Bildung einer schützenden Menge von im Kreislauf zirkulierenden Antikörpern zu führen.

30. Zusammensetzung zum Diagnostizieren einer Malaria-Infektion in einer Blutserumprobe, welche Blutserumprobe eine Zusammensetzung enthält, welche Zusammensetzung ihrerseits wenigstens eine der DNA-Sequenzen nach einem der Ansprüche 1 bis 16 oder wenigstens eines der Polypeptide nach den Ansprüchen 21 oder 22 und/oder wenigstens einen Antikörper gegen ein Polypeptid nach den Ansprüchen 21 oder 22 enthält.

31. Verfahren zum Diagnostizieren einer Malaria-Infektion, welches die Stufe des Prüfens einer Blutserumprobe auf das Vorliegen einer Zusammensetzung umfaßt, welche die DNA-Sequenzen nach einem der Ansprüche 1 bis 16 oder wenigstens eines der Polypeptide nach den Ansprüchen 21 oder 22 oder wenigstens einen Antikörper gegen ein Polypeptid nach den Ansprüchen 21 oder 22 enthält.

## Revendications

1. Séquence d'ADN codant lors de l'expression pour un antigène paludéen de stade schizonte-mérozoïte tardif, spécifique de stade, conférant à des primates l'immunité au paludisme, laquelle séquence d'ADN est choisie parmi des séquences d'ADN qui s'hybrident, dans des conditions de sels et de température correspondant à 2 × SCC et 65°C, avec une séquence d'ADN enrichie en ADN de stade de schizonte-mérozoïte tardif, mais ne s'hybrident pas, dans ces conditions, avec une séquence d'ADN enrichie en ADN de corps annulaire, lesdits enrichissements d'ADN étant apportés par un procédé comprenant les étapes de culture de globules rouges parasités par une espèce de *Plasmodium,* jusqu'à ce qu'ils contiennent plus de 80 % de schizontes, purification des schizontes à partir de la culture, infestation avec les schizontes purifiés de globules rouges non infestés, pendant une période d'environ 6 heures, lyse des formes matures restantes, dispersion et culture des cellules, collecte de l'ARN enrichi en ARN de corps annulaire, à partir d'une première partie aliquote des cellules après 6 heures environ, collecte de l'ARN enrichi en ARN de stade schizonte-mérozoïte tardif, à partir d'une seconde partie aliquote des cellules après environ 33 heures, et transcription inverse desdits ARN en ADNc; et des séquences d'ADN qui s'hybrident, dans des conditions de sels et de température correspondant à 2 × SCC et 65°C, avec lesdites séquences d'ADN; et des séquences d'ADN qui codent, lors de l'expression, pour un polypeptide codé, lors de l'expression, par l'une quelconque des séquences d'ADN précédentes.

2. Séquence d'ADN selon la revendication 1, dans laquelle ledit antigène paludéen est choisi parmi les antigènes de *P. falciparum, P. vivax, P. malariae* et *P. ovale.*

3. Séquence d'ADN choisie parmi les segments d'insertion d'ADN de pBR322(Pst)/V14 désigné par le numéro de dépôt DSM 2888, des séquences d'ADN qui s'hybrident avec lesdits segments d'insertion d'ADN dans des conditions de sels et de température correspondant à 2 × SCC et 65°C, et des séquences d'ADN qui codent, lors de l'expression, pour un polypeptide codé, lors de l'expression, par l'un quelconque des segments d'insertion d'ADN précédents ou l'une quelconque des séquences d'ADN précédentes.

4. Séquence d'ADN choisie parmi les segments d'insertion d'ADN de pBR322(Pst)/G2 désigné par le numéro de dépôt DSM 2879, des séquences d'ADN qui s'hybrident avec lesdits segments d'insertion d'ADN dans des conditions de sels et de température correspondant à 2 × SCC et 65°C, et des séquences d'ADN qui codent, lors de l'expression, pour un polypeptide codé, lors de l'expression, par l'un quelconque des segments d'insertion d'ADN précédents ou l'une quelconque des séquences d'ADN précédentes.

5. Séquence d'ADN choisie parmi les segments d'insertion d'ADN de pBR322(Pst)/R14 désigné par le numéro de dépôt DSM 2886, des séquences d'ADN qui s'hybrident avec lesdits segments d'insertion d'ADN dans des conditions de sels et de température correspondant à 2 × SCC et 65°C, et des séquences d'ADN qui codent, lors de l'expression, pour un polypeptide codé, lors de l'expression, par l'un quelconque des segments d'insertion d'ADN précédents ou l'une quelconque des séquences d'ADN précédentes.

6. Séquence d'ADN choisie parmi les segments d'insertion d'ADN de pBR322(Pst)/D4 désigné par le numéro de dépôt DSM 2887, des séquences d'ADN qui s'hybrident avec lesdits segments d'insertion d'ADN dans des conditions de sels et de température correspondant à 2 × SCC et 65°C, et des séquences d'ADN qui codent, lors de l'expression, pour un polypeptide codé, lors de l'expression, par l'un quelconque des segments d'insertion d'ADN précédents ou l'une quelconque des séquences d'ADN précédentes.

7. Séquence d'ADN choisie parmi les segments d'insertion d'ADN de pBR322(Pst)/X11 désigné par le numéro de dépôt DSM 2882, des séquences d'ADN qui s'hybrident avec lesdits segments d'insertion d'ADN dans des conditions de sels et de température correspondant à 2 × SCC et 65°C, et des séquences d'ADN qui codent, lors de l'expression, pour un polypeptide codé, lors de l'expression, par l'un quelconque des segments d'insertion d'ADN précédents ou l'une quelconque des séquences d'ADN précédentes.

8. Séquence d'ADN choisie parmi les segments d'insertion d'ADN de pBR322(Pst)/M2 désigné par le numéro de dépôt DSM 2883, des séquences d'ADN qui s'hybrident avec lesdits segments d'insertion d'ADN dans des conditions de sels et de température correspondant à 2 × SCC et 65°C, et des séquences d'ADN qui codent, lors de l'expression, pour un polypeptide codé, lors de l'expression, par l'un quelconque des segments d'insertion d'ADN précédents ou l'une quelconque des séquences d'ADN précédentes.

9. Séquence d'ADN choisie parmi les segments d'insertion d'ADN de pBR322(Pst)/T5 désigné par le numéro de dépôt DSM 2889, des séquences d'ADN qui s'hybrident avec lesdits segments d'insertion d'ADN dans des conditions de sels et de température correspondant à 2 × SCC et 65°C, et des séquences d'ADN qui codent, lors de l'expression, pour un polypeptide codé, lors de l'expression, par l'un quelconque des segments d'insertion d'ADN précédents ou l'une quelconque des séquences d'ADN précédentes.

10. Séquence d'ADN choisie parmi les segments d'insertion d'ADN de pBR322(Pst)/M7 désigné par le numéro de dépôt DSM 2884, des séquences d'ADN qui s'hybrident avec lesdits segments d'insertion d'ADN dans des conditions de sels et de température correspondant à 2 × SCC et 65°C, et des séquences d'ADN qui codent, lors de l'expression, pour un polypeptide codé, lors de l'expression, par l'un quelconque des segments d'insertion d'ADN précédents ou l'une quelconque des séquences d'ADN précédentes.

11. Séquence d'ADN choisie parmi les segments d'insertion d'ADN de pBR322(Pst)/V4 désigné par le numéro de dépôt DSM 2880, des séquences d'ADN qui s'hybrident avec lesdits segments d'insertion d'ADN dans des conditions de sels et de température correspondant à 2 × SCC et 65°C, et des séquences d'ADN qui codent, lors de l'expression, pour un polypeptide codé, lors de l'expression, par l'un quelconque des segments d'insertion d'ADN précédents ou l'une quelconque des séquences d'ADN précédentes.

12. Séquence d'ADN choisie parmi les segments d'insertion d'ADN de pBR322(Pst)/D8 désigné par le numéro de dépôt DSM 2881, des séquences d'ADN qui s'hybrident avec lesdits segments d'insertion d'ADN dans des conditions de sels et de température correspondant à 2 × SCC et 65°C, et des séquences d'ADN qui codent, lors de l'expression, pour un polypeptide codé, lors de l'expression, par l'un quelconque des segments d'insertion d'ADN précédents ou l'une quelconque des séquences d'ADN précédentes.

13. Séquence d'ADN choisie parmi les segments d'insertion d'ADN de pBR322(Pst)/14 désigné par le numéro de dépôt DSM 2878, des séquences d'ADN qui s'hybrident avec lesdits segments d'insertion d'ADN dans des conditions de sels et de température correspondant à 2 × SCC et 65°C, et des séquences d'ADN qui codent, lors de l'expression, pour un polypeptide codé, lors de l'expression, par l'un quelconque des segments d'insertion d'ADN précédents ou l'une quelconque des séquences d'ADN précédentes.

14. Séquence d'ADN choisie parmi les segments d'insertion d'ADN de pBR322(Pst)/E8 désigné par le numéro de dépôt DSM 2885, des séquences d'ADN qui s'hybrident avec lesdits segments d'insertion d'ADN dans des conditions de sels et de température correspondant à 2 × SCC et 65°C, et des séquences d'ADN qui codent, lors de l'expression, pour un polypeptide codé, lors de l'expression, par l'un quelconque des segments d'insertion d'ADN précédents ou l'une quelconque des séquences d'ADN précédentes.

15. Séquence d'ADN choisie parmi les segments d'insertion d'ADN de PPL31A-Pst(Pst)1 désigné par le numéro de dépôt DSM 2891, PPL31A-Pst(Pst)X11-011 désigné par le numéro de dépôt DSM 2890, des séquences d'ADN qui s'hybrident avec lesdits segments d'insertion d'ADN dans des conditions de sels et de température correspondant à 2 × SCC et 65°C, et des séquences d'ADN qui codent, lors de l'expression, pour un polypeptide codé, lors de l'expression, par l'un quelconque des segments d'insertion d'ADN précédents ou l'une quelconque des séquences d'ADN précédentes.

16. Séquence d'ADN selon la revendication 15, choisie parmi le segment d'insertion paludéen de pFCC-1 tel qu'indiqué sur la figure 6 et désigné par le numéro de dépôt ATCC 53035, des séquences d'ADN qui s'hybrident avec ledit segment d'insertion d'ADN dans des conditions de sels et de température correspondant à 2 × SCC et 65°C, et des séquences d'ADN qui codent, lors de l'expression, pour un polypeptide codé, lors de l'expression, par l'un quelconque des segments d'insertion d'ADN précédents ou l'une quelconque des séquences d'ADN précédentes.

17. Molécule d'ADN recombinant comprenant une séquence d'ADN choisie parmi les séquences d'ADN de l'une quelconque des revendications 1 à 16.

18. Molécule d'ADN recombinant selon la revendication 17, caractérisée en ce que ladite molécule comprend en outre une séquence régulatrice d'expression, ladite séquence régulatrice d'expression étant fonctionnellement liée à ladite séquence d'ADN de l'une quelconque des revendications 1 à 16.

19. Hôte transformé par au moins une molécule d'ADN recombinant choisie parmi les molécules d'ADN recombinant de la revendication 17 ou 18.

20. Hôte selon la revendication 19, choisi parmi *E. coli, Bacillus, Pseudomonas*, des levures, d'autres champignons, *Streptomyces,* des cellules de souris ou d'autres vertébrés, des cellules d'insectes, des cellules végétales et des cellules de tissus humains.

21. Polypeptide codé, lors de d'expression, par une séquence d'ADN choisie parmi des séquences d'ADN de l'une quelconque des revendications 3 à 16.

22. Polypeptide produit par un procédé de culture d'un hôte transformé par une molécule d'ADN recombinant choisie parmi les molécules d'ADN des revendications 3 à 16, dans lequel lesdites molécules comprennent en outre une séquence régulatrice d'expression, ladite séquence régulatrice d'expression étant fonctionnellement liée auxdites molécules.

23. Procédé pour la sélection d'une séquence d'ADN codant, lors de l'expression, pour un antigène paludéen de stade de schizonte-mérozoïte tardif, spécifique de stade, parmi des séquences d'ADN, comprenant l'étape qui consiste à déterminer laquelle desdites séquences s'hybride, dans des conditions de sels et de température correspondant à 2 × SCC et 65°C, avec une séquence d'ADN choisie parmi les séquences d'ADN de l'une quelconque des revendications 1 à 16.

**24.** Procédé pour la sélection d'une séquence d'ADN codant, lors de l'expression, pour un antigène paludéen de stade de schizonte-mérozoïte tardif, spécifique de stade, et conférant à des primates une immunité contre le paludisme, à partir d'un groupe de séquences d'ADN, comprenant l'étape qui consiste à déterminer laquelle desdites séquences d'ADN s'hybride, dans des conditions de sels et de température correspondant à 2 × SCC et 65°C, avec une séquence d'ADN enrichie en ADN de stade de schizonte-mérozoïte tardif, mais ne s'hybride pas, dans ces conditions, à une séquence d'ADN enrichie en ADN de corps annulaire, lesdits enrichissements en ADN étant produits par un procédé comprenant les étapes de culture de globules rouges parasités par une espèce de *Plasmodium*, jusqu'à ce qu'ils contiennent plus de 80 % de schizontes, purification des schizontes à partir de la culture, infestation de globules rouges non infestés avec les schizontes purifiés, pendant une période d'environ 6 heures, lyse de la forme mature restante, dispersion et culture des cellules, collecte d'ARN enrichi en ARN de corps annulaire, à partir d'une première partie aliquote des cellules après 6 heures environ, récolte d'ARN enrichi en ARN de stade schizonte-mérozoïte tardif, à partir d'une seconde partie aliquote des cellules au bout d'environ 33 heures, et transcription inverse desdits ARN en ADNc.

**25.** Procédé selon la revendication 23 ou 24, dans lequel ledit groupe de séquences d'ADN est choisi parmi des séquences d'ADN provenant de sources naturelles, synthétiques ou semi-synthétiques.

**26.** Procédé selon l'une quelconque des revendications 23 à 25, dans lequel ledit antigène paludéen est choisi parmi les antigènes de *P. falciparum, P. vivax, P. malariae* et *P. ovale.*

**27.** Procédé pour la production d'un antigène de stade de schizonte-mérozoïte tardif, spécifique de stade, comprenant les étapes de culture d'un hôte selon la revendication 19 et de collecte de l'antigène.

**28.** Procédé selon la revendication 27, dans lequel ledit hôte est choisi parmi *E. coli, Bacillus, Pseudomonas,* des levures, d'autres champignons, *Streptomyces,* des cellules de souris ou d'autres vertébrés, des cellules d'insectes, des cellules végétales et des cellules de tissus humains.

**29.** Composition pharmaceutiquement acceptable pour la protection contre des infestations paludéennes, comprenant une quantité d'au moins un polypeptide choisi parmi les polypeptides de la revendication 21 ou 22, efficaces pour induire un taux protecteur d'anticorps circulants lors de la libération de mérozoïtes par des globules rouges.

**30.** Composition pour le diagnostic d'une infestation paludéenne dans un échantillon de sérum sanguin, comprenant une composition qui contient au moins l'une des séquences d'ADN de l'une quelconque des revendications 1 à 16 ou au moins l'un des polypeptides de la revendication 21 ou 22 et/ou au moins un anticorps dirigé contre un polypeptide de la revendication 21 ou 22.

**31.** Procédé pour le diagnostic d'une infestation paludéenne, comprenant l'étape d'essai d'un échantillon de sérum sanguin pour la détermination de la présence d'une composition qui contient les séquences d'ADN de l'une quelconque des revendications 1 à 16 ou au moins l'un des polypeptides de la revendication 21 ou 22 ou au moins un anticorps dirigé contre un polypeptide de la revendication 21 ou 22.

# FIG.1

## FIG.2

FIG.3

# FIG.4

```
GlyGlyGlyGlyGlyTyrGlnGluLeuValLysLysLeuGluAlaLeuGluAspAlaVal
GGGGGGGGGGGGGGGGGGTTATCAAGAACTTGTCAAAAAACTAGAAGCTTTAGAAGATGCAC

LeuThrGlyTyrSerLeuPheGlnLysGluLysMetValLeuAsnGluGlyThrSerGly
TATTGACAGGTTATAGTTTATTTCAAAAGGAAAAAATGGTATTAAATGAAGGAACAAGTG

ThrAlaValThrThrSerThrProGlySerGlyGlySerValThrSerGlyGlySerGly
GAACAGCTGTTACAACTAGTACACCTGGTTCAGGTGGTTCAGTTACTTCAGGTGGTTCAG

GlySerValAlaSerValAlaSerGlyGlySerGlyGlySerValAlaSerGlyGlySer
GTGGTTCAGTTGCTTCAGTTGCTTCAGGTGGTTCAGGTGGCTCAGTTGCTTCAGGTGGTT

GlyAsnSerArgArgThrAsnProSerAspAsnSerSerAspSerAspAlaLysSerSer
CAGGTAATTCAAGACGTACAAATCCTTCAGATAATTCAAGTGATTCAGATGCTAAATCTT

ProArg
CCCCCC
```

Fig. 5

```
       C   R   G   G   G   G   G   T   Y   V   *   G   K   L   C   E   *   Y   *   N   Y
   1   CCTGCAGGGGGGGGGGGGGTACATATGTGTAAGGAAAATTATGTGAATAATATTAAAATT   60

       S   Y   D   V   I   N   N   F   Y   Y   K   N   K   A   N   V   K   C   K   N
  61   ATAGTTATGATGTAATAAATAATTTTTATTATAAAAATAAGGCTAATGTAAAATGCAAAA   120

       K   C   I   H   I   F   A   K   S   Y   F   *   I   I   N   L   F   Y   Y   Y
 121   ATAAATGTATACATATTTTTGCTAAGTCATATTTTTAAATTATTAACTTATTTTATTATT   180

       Y   F   Y   L   Y   I   L   F   I   S   F   N   S   I   M   K   I   I   F   F
 181   ATTATTTTTATTTATATATATTATTTATTAGCTTTAATTCAATAATGAAGATCATATTCT   240

       L   C   S   F   L   F   F   I   I   N   T   Q   C   V   T   H   E   S   Y   Q
 241   TTTTATGTTCATTTCTTTTTTTTTATTATAAATACACAATGTGTAACACATGAAAGTTATC   300

       E   L   V   K   K   L   E   A   L   E   D   A   V   L   T   G   Y   S   L   F
 301   AAGAACTTGTCAAAAAACTAGAAGCTTTAGAAGATGCAGTATTGACAGGTTATAGTTTAT   360

       Q   K   E   K   M   V   L   N   E   G   T   S   G   T   A   V   T   T   S   T
 361   TTCAAAAGGAAAAAATGGTATTAAATGAAGGAACAAGTGGAACAGCTGTTACAACTAGTA   420

       P   G   S   G   G   S   V   T   S   G   G   S   G   G   S   V   A   S   V   A
 421   CACCTGGTTCAGGTGGTTCAGTTACTTCAGGTGGTTCAGGTGGTTCAGTTGCTTCAGTTG   480

       S   G   S   G   G   S   V   A   S   G   G   S   G   N   S   R   R   T   N
 481   CTTCAGGTGGTTCAGGTGGCTCAGTTGCTTCAGGTGGTTCAGGTAATTCAAGACGTACAA   540

       P   S   D   N   S   S   D   S   D   A   K   S   Y   A   D   L   K   H   R   V
 541   ATCCTTCAGATAATTCAAGTGATTCAGATGCTAAATCTTACGCTGATTTAAAACATAGAG   600

       Q   N   Y   L   F   T   I   K   E   L   K   Y   P   E   L   F   D   L   T   N
 601   TTCAAAATTACTTGTTCACTATTAAAGAACTCAAATATCCCGAACTCTTTGATTTAACCA   660

       H   N   L   T   L   C   D   N   I   H   G   F   K   Y   L   I   D   G   Y   E
 661   ATCATATGTTAACTTTGTGTGATAATATTCATGGTTTCAAATATTTAATTGATGGATATG   720

       E   I   T   P   ?
 721   AAGAAATTACCCCCCCC
```

Fig. 6

30